(19) Europäisches Patentamt
European Patent Office
Office européen des brevets

(11) EP 1 234 834 A1

(12) **EUROPEAN PATENT APPLICATION**
published in accordance with Art. 158(3) EPC

(43) Date of publication:
**28.08.2002 Bulletin 2002/35**

(21) Application number: **00978052.9**

(22) Date of filing: **01.12.2000**

(51) Int Cl.7: **C07K 5/083**, A61K 38/06, A61P 1/02, A61P 1/04, A61P 9/10, A61P 11/00, A61P 29/00, A61P 43/00

(86) International application number:
**PCT/JP00/08516**

(87) International publication number:
**WO 01/040263 (07.06.2001 Gazette 2001/23)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **03.12.1999 JP 34445099**

(71) Applicant: **ONO PHARMACEUTICAL CO., LTD.**
**Osaka-shi, Osaka 541-8526 (JP)**

(72) Inventors:
- **OHMOTO, Kazuyuk, Minase Research Institute Shimamoto-cho, Mishima-gun Osaka 618-858 (JP)**
- **OKUMA, Motohiro, Minase Research Institute Shimamoto-cho, Mishima-gun Osaka 618-858 (JP)**
- **SEKIOKA, Tomohiko, Minase Research Institute Shimamoto-cho, Mishima-gun Osaka 618-858 (JP)**

(74) Representative: **Henkel, Feiler, Hänzel**
**Möhlstrasse 37**
**81675 München (DE)**

(54) **1,3,4-OXADIAZOLIN-2-ONE DERIVATIVES AND DRUGS CONTAINING THESE DERIVATIVES AS THE ACTIVE INGREDIENT**

(57)

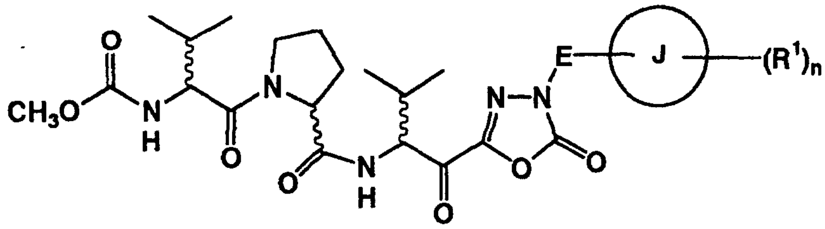

(I)

The compounds of formula (I) have an elastase inhibitory activity, therefor, they are useful for the treatment and/ or prevention of a disease induced by an abnormal enhancement of degradation of elastin, collagen fiber and / or proteoglycans by elastase, for example, pulmonary emphysema, rheumatoid arthritis, arteriosclerosis, adult respiratory distress syndrome, myocardial infarction, ulcerative colitis and gingivitis.

EP 1 234 834 A1

## Description

Technical Field

This invention relates to 1, 3, 4-oxadiazolin-2-one derivatives. More particularly, this invention relates to:

(1) 1,3,4-oxadiazolin-2-one derivatives of formula (I)

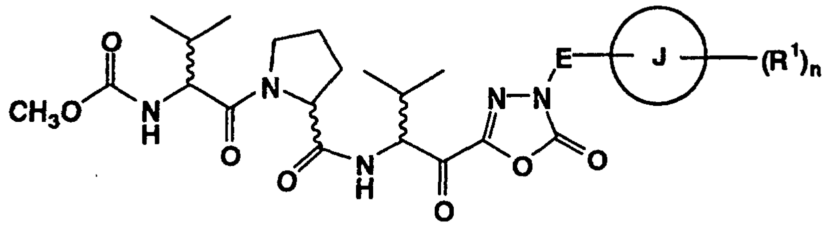

(I)

wherein all the symbols are as hereinafter defined;
or a non-toxic salt thereof,
(2) a process for the preparation thereof and
(3) a pharmaceutical composition comprising them as active ingredient.

Background

Recently, researches and developments concerning elastase inhibitors are becoming active.

For example, (1) the compound of formula (X)

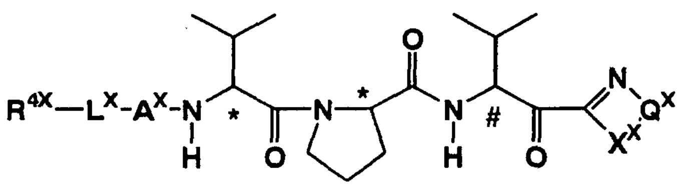

(X)

or a non-toxic salt thereof or an acid addition salt thereof was disclosed to have an elastase inhibitory activity (JP-A-64-45395; EP 291234). As a representative example, the compound of formula (X-1)

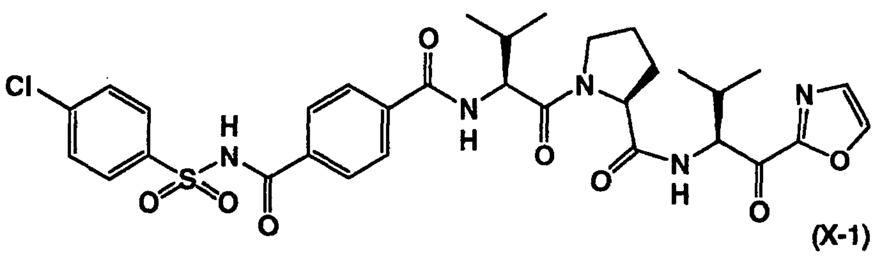

(X-1)

was disclosed.
(2) the compound of formula (Y)

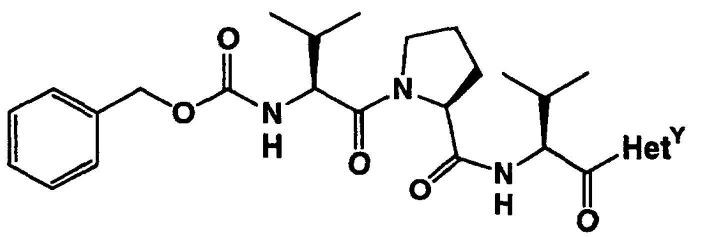 (Y)

was disclosed to have an elastase inhibitory activity [J. Med. Chem. 38, 76 (1995)]. As a representative example, the compound of formula (Y-1)

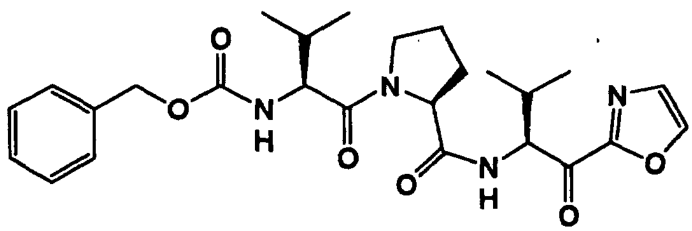 (Y-1)

was disclosed.

(3) the compounds of formula (Z-a) - (Z-f)

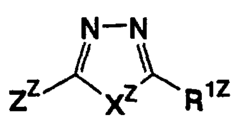 (Z-a)

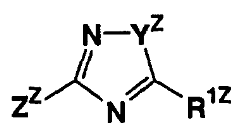 (Z-b)

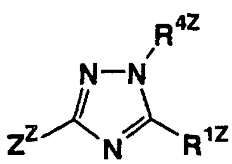 (Z-c)

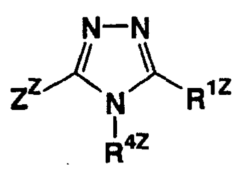 (Z-d)

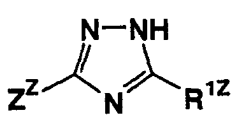 (Z-e)

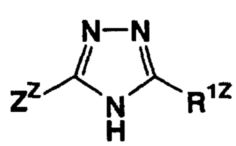 (Z-f)

were disclosed to have a serine protease, especially, an elastase inhibitory activity (WO 96/16080). As a representative example, the compound of formula (Z-1)

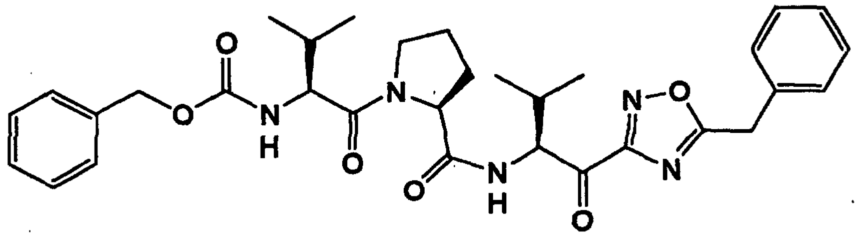

(Z-1)

was disclosed.

(4) the compounds of formula (W)

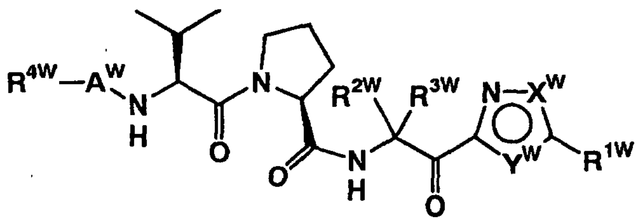

(W-a)

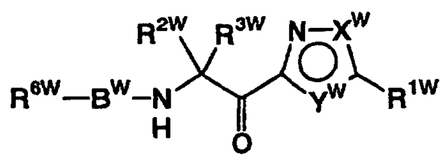

(W-b)

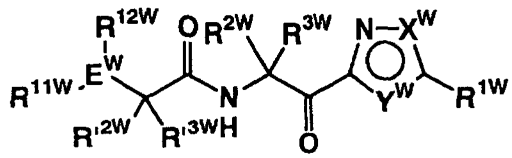

(W-c)

were disclosed to have a serine protease, especially, an elastase inhibitory activity (WO 98/24806). As representative examples, the compounds of formula (W-1) and (W-2)

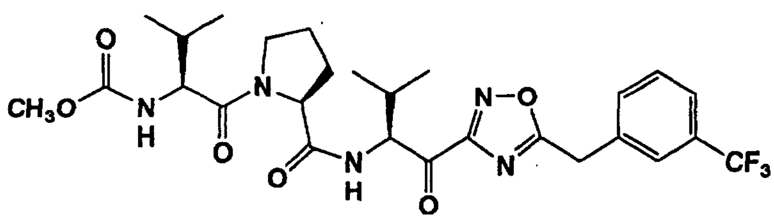

(W-1)

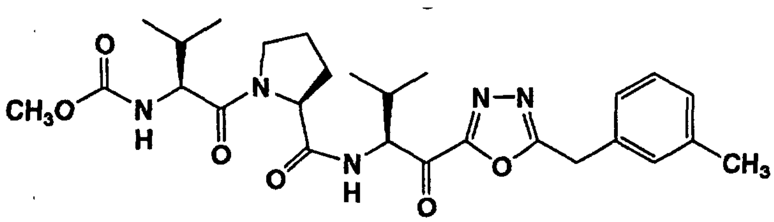

(W-2)

were disclosed.

In hitherto known elastase inhibitors, only a few compound was confirmed to have effect when orally administered and so they are not expected the effect by oral administration. If a pharmaceutical agent is able to do effect when orally administered, it is fundamental that an absorption from gastrointestinal is good and the activity can be sustained until a pharmaceutical agent come to an active site. Therefore, a compound having a superior stability, absorption and solubility at an active site is considered to do effect enough when orally administered.

Disclosure of the invention

Energetic investigations have been carried out in order to find the compound having an excellent elastase inhibitory activity and a high safety. The present inventors have found 1, 3, 4-oxadiazolin-2-one derivatives of formula (I) and accomplished the present invention.
1, 3, 4-oxadiazolin-2-one derivatives of formula (I) or a non-toxic salt thereof are novel compound.
Besides, the present inventors have found the compounds that had a superior stability, absorption and solubility, and showed an inhibitory activity on elastase by oral administration.
This invention relates to:

(1) 1,3,4-oxadiazolin-2-one derivatives of formula (I)

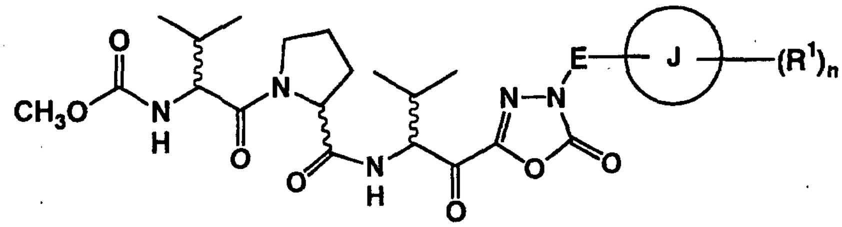

(I)

wherein E is a single bond or C1-8 alkylene,
J is C5-15 mono-, bi- or tri-carbacyclic ring or 5-18 membered mono-, bi- or tri-heterocyclic ring containing 1-4 of nitrogen, 1-2 of oxygen and/or 1-2 sulfur,
$R^1$ is

(1) C1-8 alkyl,
(2) halogen atom,
(3) nitro,
(4) trifluoromethyl,
(5) trifluoromethoxy,
(6) cyano,
(7) phenyl,
(8) tetrazolyl,
(9) $-NR^2R^3$,
(10) $-OR^4$,
(11) $-SR^5$,
(12) $-COR^6$ or
(13) C1-8 alkyl substituted by halogen atom, nitro, trifluoromethyl, trifluoromethoxy, cyano, phenyl, tetrazolyl, $-NR^2R^3$, $-OR^4$, $-SR^5$ or $-COR^6$, in which $R^2$, $R^3$, $R^4$ and $R^5$ each, independently, is hydrogen, C1-4 alkyl, phenyl or C1-4 alkyl substituted by phenyl, $R^6$ is C1-4 alkyl, phenyl, C1-4 alkyl substituted by phenyl, $-NR^2R^3$ or $-OR^4$,
n is 0 or 1-5;
or a non-toxic salt thereof,

(2) a process for the preparation thereof,
(3) a pharmaceutical composition comprising them as active ingredient.

Detailed description of Invention

In the compound of formula (I), C1-8 alkylene represented by E means methylene, ethylene, trimethylene, tetramethylene, pentamethylene, hexamethylene, heptamethylene, octamethylene and isomeric groups thereof.
In the compound of formula (I), C1-8 alkyl represented by $R^1$ means methyl, ethyl, propyl, butyl, pentyl, hexyl, heptyl, octyl and isomeric groups thereof.
In the compound of formula (I), halogen atom represented by $R^1$ is fluorine, chlorine, bromine and iodine.
In the compound of formula (I), C1-8 alkyl substituted by halogen atom, nitro, trifluoromethyl, trifluoromethoxy, cyano, phenyl, tetrazolyl, $-NR^2R^3$, $-OR^4$, $-SR^4$ or $-COR^6$ represented by $R^1$ means methyl, ethyl, propyl, butyl, pentyl, hexyl, heptyl, octyl and isomeric groups thereof substituted by 1 of halogen atom, nitro, trifluoromethyl, trifluoromethoxy, cyano, phenyl, tetrazolyl, $-NR^2R^3$, $-OR^4$, $-SR^4$ or $-COR^6$.
In the compound of formula (I), C1-4 alkyl represented by $R^2$, $R^3$, $R^4$, $R^5$ and $R^6$ means methyl, ethyl, propyl, butyl and isomeric groups thereof.
In the compound of formula (I), C1-4 alkyl substituted by phenyl represented by $R^2$, $R^3$, $R^4$, $R^5$ and $R^6$ means methyl, ethyl, propyl, butyl and isomeric groups thereof substituted by 1 of phenyl.
In the compound of formula (I), C5-15 mono-, bi-, or tri-carbocyclic ring represented by J is C5-15 mono-, bi-, or tri-carbocyclic aryl or partially saturated thereof. For example, cyclopentadiene, benzene, indene, pentalene, naphthalene, fluorene, anthracene, dihydroindene, dihydronaphthalene, tetrahydronaphthalene, dihydrofluorene, tetrahydrofluorene, dihydroanthracene, tetrahydroanthracene.
In the compound of formula (I), 5-18 membered mono-, bi-, or tri-heterocyclic ring containing 1-4 of nitrogen, 1-2 of oxygen and/or 1-2 of sulfur is 5-18 membered mono-, bi-, or tri-heterocyclic aryl or partially saturated thereof containing 1-4 of nitrogen, 1-2 of oxygen and/or 1-2 of sulfur. For example, pyrrole, imidazole, pyrazole, triazole, tetrazole, pyridine, pyrazine, pyrimidine, pyridazine, azepine, diazepine, furan, pyran, oxepin, oxazepine, thiophene, thiain (thiopyran), thiepin, oxazole, isoxazole, thiazole, isothiazole, oxadiazole, oxazine, oxadiazine, oxazepine, oxadiazepine, thiadiazole, thiazine, thiadiazine, thiazepine, thiadiazepine, indole, isoindole, benzofuran, isobenzofuran, benzothiophene, isobenzothiophene, indazole, quinoline, isoquinoline, phthalazine, naphthyridine, quinoxaline, quinazoline, cinnoline, benzoxazole, benzothiazole, benzoimidazole, carbazole, acridine, dibenzofuran, dibenzothiophene, indoline, isoindoline, dihydrobenzofuran, dihydroisobenzofuran, dihydrobenzothiophene, dihydroisobenzothiophene, dihydroindazole, dihydroquinoline, tetrahydroquinoline, dihydroisoquinoline, tetrahydroisoquinoline, dihydrophthalazine, tetrahydrophthalazine, dihydronaphthyridine, tetrahydronaphthyridine, dihydroquinoxaline, tetrahydroquinoxaline, dihydroquinazoline, tetrahydroquinazoline, dihydrocinnoline, tetrahydrocinnoline, dihydrobenzoxazole, dihydrobenzothiazole, dihydrobenzimidazole, benzoxazepine, benzoxadiazepine, benzothiazepine, benzothiadiazepine, benzazepine, benzodiazepine, indoloxoazepine, indolotetrahydrooxazepine, indoloxadiazepine, indolotetrahydrooxadiazepine, indolothiazepine, indolotetrahydrothiazepine, indolothiadiazepine, indolotetrahydrothiadiazepine, indoloazepine, indolotetrahydroazepine, indolodiazepine, indolotetrahydrodiazepine, benzofurazan, benzothiadiazole, benzotriazole, imidazothiazole, dihydrocarbazole, tetrahydrocarbazole, dihydroacridine, tetrahydroacridine, dihydrodibenzofuran, dihydrodibenzothiophene, tetrahydrodibenzofuran, tetrahydrodibenzothiophene, 1,3-dioxaindan, 1,4-dioxotetrahydronaphthalene.
Unless otherwise specified, all isomers are included in the present invention. For example, alkyl, alkoxy and alkylene include straight and branched isomers. Isomers based on double bond, ring, fused ring (E, Z, cis, trans), isomers resulting from the presence of asymmetric carbon(s) (R-configuration, S-configuration,$\alpha$-configuration, $\beta$-configuration, enantiomers, diastereoisomers), optically active compounds having optical rotation (D, L, d, 1-configuration), polar compounds obtained by chromatographic separations (highly polar compound, less polar compound), equilibrium compounds, the mixtures are existed by free ratio, racemic mixtures are included in the present invention.
In the invention, as is apparent to one skilled in the art, unless otherwise indicated, the mark:

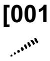

shows that the bond is on the other side of paper ($\alpha$-configuration), the mark:

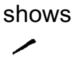

shows that the bond is in front of paper ($\beta$- configuration), the mark:

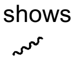

shows that the bond are $\alpha$-, $\beta$- configuration or a mixture thereof.
The compounds of formulae (I) of the present invention may be converted into the corresponding non-toxic salts by conventional means.
In the specification, non-toxic salts are salts of alkali metals, salts of alkaline earth metals, ammonium salts, amine salts, acid addition salts.
Non-toxic salts and water-soluble salts are preferred. Suitable salts, for example, include:
salts of alkali metals (e.g. potassium, sodium), salts of alkaline earth metals (e.g. calcium, magnesium), ammonium salts, salts of pharmaceutically acceptable organic amines (e.g. tetramethylammonium, triethylamine, methylamine,

dimethylamine, cyclopentylamine, benzylamine, phenethylamine, piperidine, monoethanolamine, diethanolamine, tris (hydroxymethyl)amine, lysine, arginine, N-methyl-D-glucamine).

Non-toxic acid addition salts and water-soluble acid addition salts are preferred. Suitable salts, for example, include:

salts of inorganic acids, e.g. hydrochloride, hydrobromide, hydroiodide, sulfate, phosphate, nitrate; salts of organic acids, e.g. acetate, lactate, tartarate, benzoate, citrate, methanesulphonate, ethanesulphonate, benzenesulphonate, toluenesulphonate, isethionate, glucuronate, gluconate.

The compounds of formula (I) and salts thereof may be converted into the corresponding a solvate. Non-toxic solvate and water-soluble solvate are preferred. Suitable solvate, for example, include water, alcohol e.g. ethanol.

In the compound of formula (I), preferable E is a single bond or C1-4 alkylene, and especially a single bond or methylene is preferable.

In the compound of formula (I), preferable J is C5-7 mono-carbocyclic ring or 5-7 membered mono-heterocyclic ring containing 1-4 of nitrogen, 1 of oxygen and/or 1 of sulfur, and especially benzene or thiophene is preferable.

In the compound of formula (I), preferable $R^1$ is C1-8 alkyl, $-NR^2R^3$, halogen atom or $-OR^4$, and especially methyl, dimethylamino, fluorine or methoxy is preferable.

In the compound of formula (I), preferable n is 0, 1 or 2.

In the compound of formula (I), preferable compound is 1, 3, 4-oxadiazolin-2-one derivative of formula (I-1)

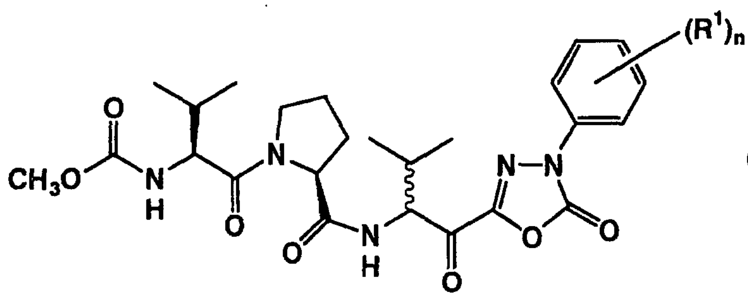

(I-1)

wherein $R^1$ and n are as hereinbefore defined;
the compound of formula (I-2)

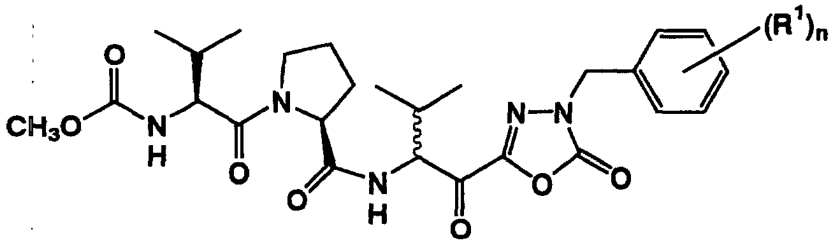

(I-2)

wherein $R^1$ and n are as hereinbefore defined;
the compound of formula (I-3)

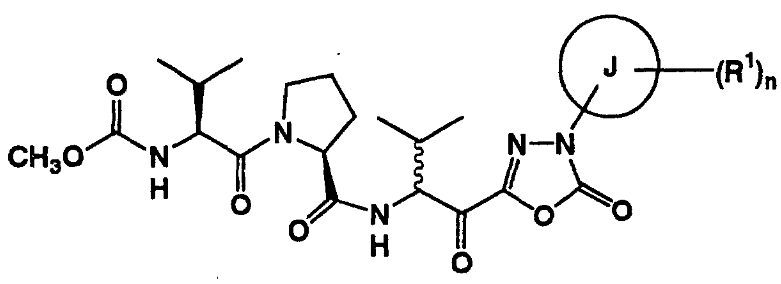

(I-3)

wherein J, $R^1$ and n are as hereinbefore defined; or

the compound of formula (I-4)

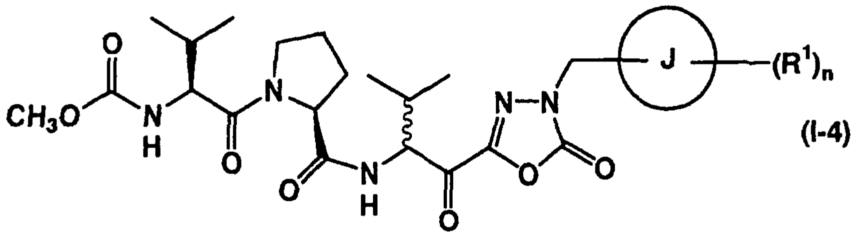

(I-4)

wherein J, $R^1$ and n are as hereinbefore defined;
or a non-toxic salt thereof.

Specific compounds in the present invention are compounds shown in Table 1-4 and described in examples, a non-toxic salt thereof, an acid addition salt thereof and a solvate thereof.

Table 1

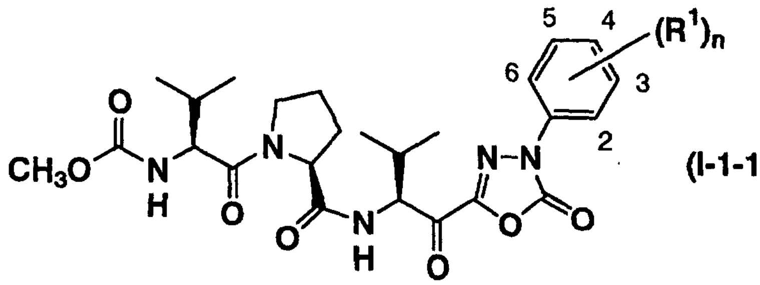

(I-1-1)

| No. | n | $R^1$ | No. | n | $R^1$ |
|---|---|---|---|---|---|
| 1 | 1 | 4-$CF_3$ | 14 | 2 | 2,6-di-F |
| 2 | 1 | 4-$NO_2$ | 15 | 2 | 3,5-di-F |
| 3 | 1 | 4-CN | 16 | 2 | 2-Cl, 4-$OCH_3$ |
| 4 | 1 | 4-$OCH_3$ | 17 | 2 | 2,6-di-$OCH_3$ |
| 5 | 1 | 4-$CH_3$ | 18 | 2 | 2,6-di-$CH_3$ |
| 6 | 1 | 4-$NH_2$ | 19 | 2 | 3,5-di-$OCH_3$ |
| 7 | 1 | 4-$N(CH_3)_2$ | 20 | 2 | 2,3-di-$CH_3$ |
| 8 | 1 | 4-$COOCH_3$ | 21 | 3 | 2,4,6-tri-Cl |
| 9 | 1 | 3-Cl | 22 | 3 | 2,4,6-tri-$OCH_3$ |
| 10 | 1 | 4-$SCH_3$ | 23 | 3 | 2,4,6-tri-$CH_3$ |
| 11 | 1 | 4-F | 24 | 1 | 3-$CH_3$ |
| 12 | 1 | 2-F | 25 | 1 | 4-$OCF_3$ |
| 13 | 1 | 2-$CH_3$ | 26 | 2 | 2,4-di-F |

Table 2

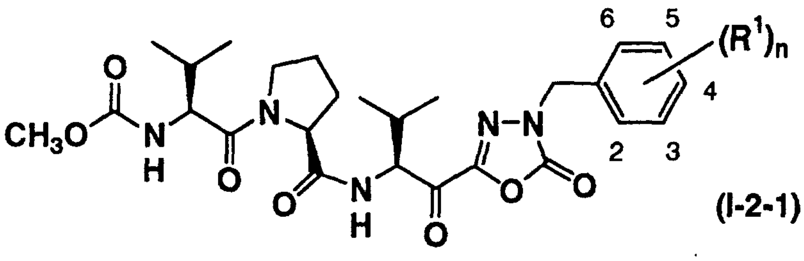

(I-2-1)

| No. | n | $R^1$ | No. | n | $R^1$ |
|---|---|---|---|---|---|
| 1 | 1 | 4-$CF_3$ | 14 | 2 | 2,6-di-F |
| 2 | 1 | 4-$NO_2$ | 15 | 2 | 3,5-di-F |
| 3 | 1 | 4-CN | 16 | 2 | 2-Cl, 4-$OCH_3$ |
| 4 | 1 | 4-$OCH_3$ | 17 | 2 | 2,6-di-$OCH_3$ |
| 5 | 1 | 4-$CH_3$ | 18 | 2 | 2,6-di-$CH_3$ |
| 6 | 1 | 4-$NH_2$ | 19 | 2 | 3,5-di-$OCH_3$ |
| 7 | 1 | 4-$N(CH_3)_2$ | 20 | 2 | 2,3-di-$CH_3$ |
| 8 | 1 | 4-$COOCH_3$ | 21 | 3 | 2,4,6-tri-Cl |
| 9 | 1 | 3-Cl | 22 | 3 | 2,4,6-tri-$OCH_3$ |
| 10 | 1 | 4-$SCH_3$ | 23 | 3 | 2,4,6-tri-$CH_3$ |
| 11 | 1 | 4-F | 24 | 1 | 3-$CH_3$ |
| 12 | 1 | 2-F | 25 | 1 | 4-$OCF_3$ |
| 13 | 1 | 2-$CH_3$ | 26 | 2 | 2,4-di-F |

Table 3

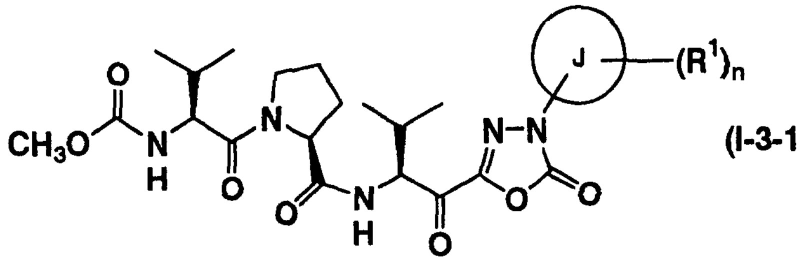

(I-3-1)

| No. | 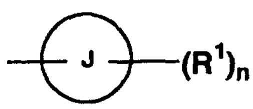 | No. | 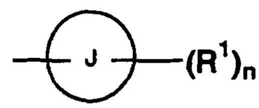 |
|---|---|---|---|
| 1 | 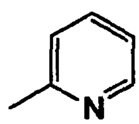 | 6 | 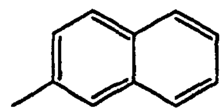 |
| 2 | 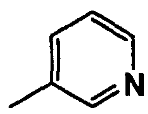 | 7 | 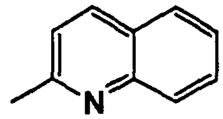 |
| 3 | 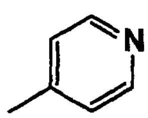 | 8 | 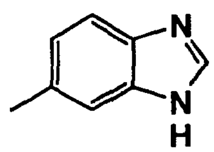 |
| 4 | 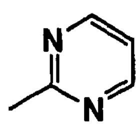 | 9 | 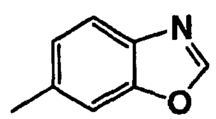 |
| 5 | 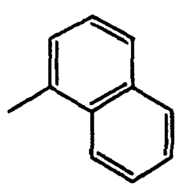 | 10 | 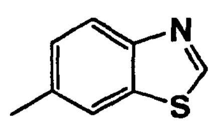 |

Table 4

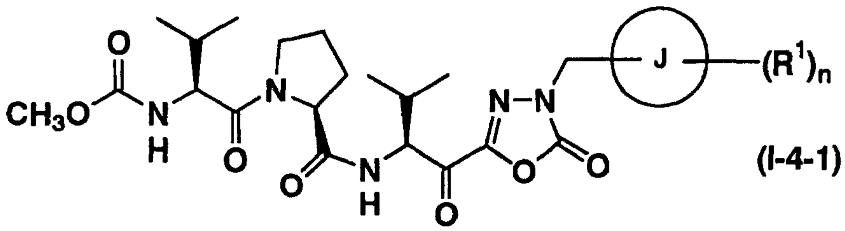

(I-4-1)

| No. | 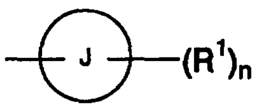 | No. | 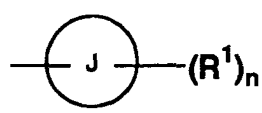 |
|---|---|---|---|
| 1 | 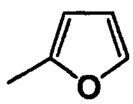 | 7 | 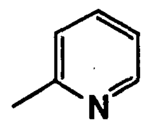 |
| 2 | 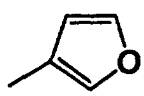 | 8 | 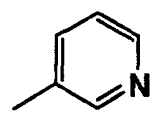 |
| 3 | 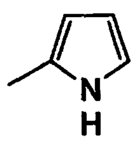 | 9 | 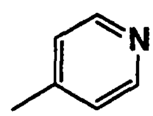 |
| 4 | 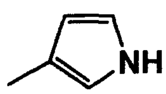 | 10 | 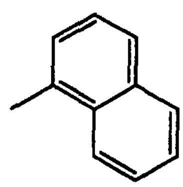 |
| 5 | 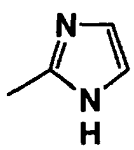 | 11 | 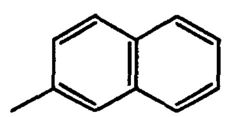 |
| 6 | 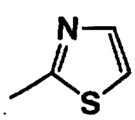 | 12 | 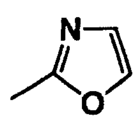 |

Process for the Preparation

In the compound of the present invention of formula (I), the compound in which both of J and $R^1$ are not groups containing carboxyl, hydroxy, amino or thiol, that is the compound of formula (IA)

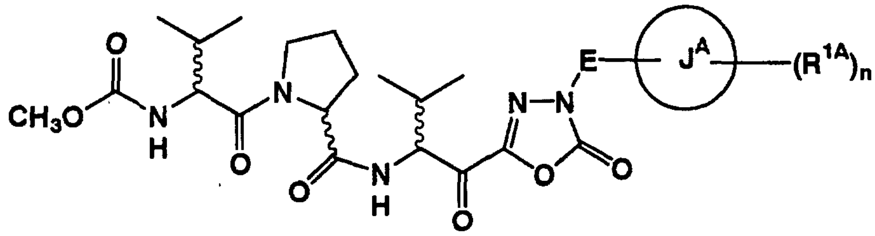

(IA)

wherein $J^A$ and $R^{1A}$ is same meaning as J and $R^1$, with the proviso that both groups are not groups containing carboxyl, hydroxy, amino or thiol, and the other symbols are as hereinbefore defined;
may be prepared by following a method of (a) or (b).

(a) The compound of formula (IA) may be prepared by subjecting the compound of formula (II)

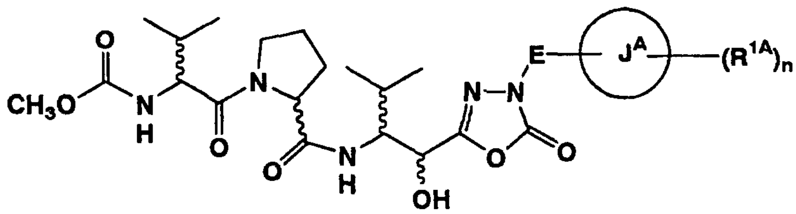

(II)

wherein all the symbols are as hereinbefore defined;
to oxidation.

The method of oxidation is known. It includes the method

(1) by Swern oxidation,
(2) using Dess-Martin Reagent,
(3) using TEMPO Reagent.

These methods are explained as follows.

(1) Swern oxidation may be carried out, for example, by reacting oxalyl chloride with dimethylsulfoxide in an organic solvent (e.g. chloroform, methylene chloride) at -78°C, and by reacting an obtained intermediate with an alcohol compound, and then by reacting an obtained compound with a tertiary amine (e.g. triethylamine) at -78-20°C.
(2) The method using Dess-Martin Reagent may be carried out, for example, in an organic solvent (e.g. chloroform, methylene chloride), using Dess-Martin Reagent (1, 1, 1-triacetoxy-1, 1-dihydro-1, 2-benziodoxol -3-(1H)-one) at 0-40°C.
(3) The method using TEMPO Reagent may be carried out, for example, in an organic solvent (e.g. chloroform, methylene chloride), using TEMPO Reagent (2, 2, 6, 6-tetramethyl-1-piperidinyloxy, free radical) at 20-60°C.

The reaction described in (1), (2) and (3) may be carried out under an inert gas (e.g. argon, nitrogen) to avoid water in order to obtain a preferable result.

As a method of oxidation, other methods, which can be oxidized alcohol to ketone easily and selectively, are also preferred, for example, Jone's oxidation, a method using pyridinium chlorochromate (PCC), a method using sulfur trioxide pyridine complex or methods described in Comprehensive Organic Transformations [Richard C. Larock, VCH Publishers, Inc., (1989) page 604-614].

(b) The compound of formula (IA) may be prepared by subjecting a compound of formula (III)

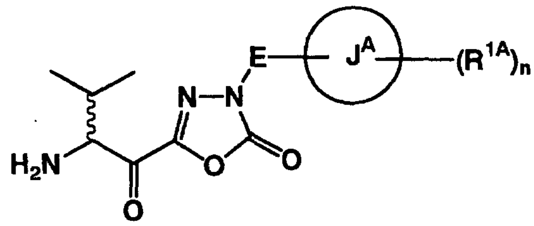

(III)

wherein all the symbols are as hereinbefore defined;
with (methoxycarbonyl)-L-valyl-L-proline of formula (IV)

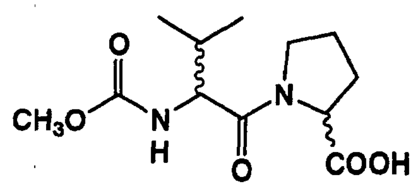

(IV)

to amidation.

The method of amidation is known. It includes the method

(1) via an acyl halide,
(2) via a mixed acid anhydride,
(3) using a condensing agent.

These methods are explained as follows.

(1) The method via an acyl halide may be carried out, for example, by reacting carboxylic acid with an acyl halide (e.g. oxalyl chloride or thionyl chloride) in an organic solvent (e.g. chloroform, methylene chloride, diethyl ether or tetrahydrofuran) or without a solvent at -20°C to reflux temperature. And then the obtained acyl halide derivative may be reacted with amine in an organic solvent (e.g. chloroform, methylene chloride, diethyl ether or tetrahydrofuran), in the presence of a tertiary amine (e.g. pyridine, triethyl amine, dimethyl aniline or dimethylaminopyridine) at 0-40°C. As an alternative, the obtained acyl halide derivative may be reacted with amine in an organic solvent (dioxane, tetrahydrofuran) using an alkaline aqueous solution (e.g. sodium bicarbonate, sodium hydroxide) at 0-40°C.

(2) The method via a mixed acid anhydride may be carried out, for example, by reacting carboxylic acid with an acyl halide (e.g. pivaloyl chloride, tosyl chloride, mesyl chloride), or an acid derivative (ethyl chloroformate or isobutyl chloroformate) in an organic solvent (e.g. chloroform, methylene chloride, diethyl ether, tetrahydrofuran) or without a solvent, in the presence of a tertiary amine (e.g. pyridine, triethylamine, dimethylaniline, dimethylaminopyridine) at 0-40°C. And then the obtained mixed acid anhydride derivative may be reacted with amine in an organic solvent (e.g. chloroform, methylene chloride, diethyl ether or tetrahydrofuran) at 0-40°C.

(3) The method using a condensing agent may be carried out, for example, by reacting carboxylic acid with amine in an organic solvent (e.g. chloroform, methylene chloride, dimethylformamide, diethyl ether or tetrahydrofuran) or without a solvent, in the presence or absence of a tertiary amine (e.g. pyridine, triethylamine, dimethylaniline or dimethylaminopyridine), using a condensing agent (e.g. 1, 3-dicyclohexyl carbodiimide (DCC), 1-ethyl-3-[3-(dimethylamino)propyl] carbodiimide (EDC), 1, 1'-carbodiimidazole (CDI), 2-chloro-1-methylpyridinium iodide), in the presence or absence of 1-hydroxybenzotiazole (HOBt) at 0-40°C.

The reaction described in (1), (2) and (3) may be carried out under an inert gas (e.g. argon, nitrogen) to avoid water in order to obtain a preferable result.

In the compound of the present invention of formula (I), the compound in which at least one of J and $R^1$ is a group containing carboxyl, hydroxy, amino or thiol, that is the compound of formula (IB)

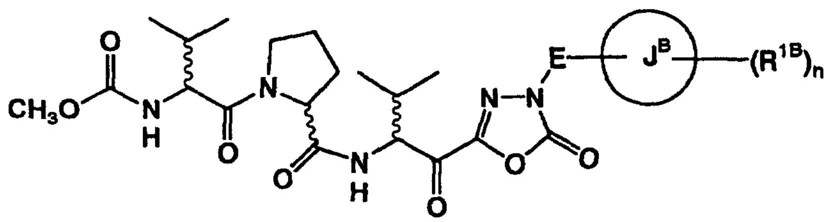

(IB)

wherein $J^B$ and $R^{1B}$ are same meaning as J and $R^1$, with the proviso that at least one of them is a group containing carboxyl, hydroxy, amino or thiol, and the other symbols are as hereinbefore defined;
may be prepared by subjecting the above compound of formula (IA) in which at least one of J and $R^1$ is a group containing protected carboxyl, hydroxy, amino or thiol by a protecting group, that is the compound of formula (IA-1)

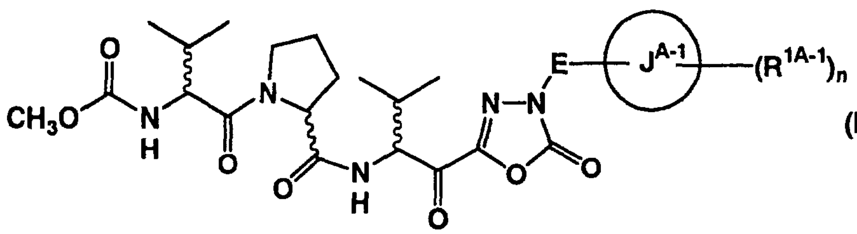

(IA-1)

wherein $J^{A-1}$ and $R^{1A-1}$ are same meaning as J and $R^1$, with the proviso that at least one of them is a group containing protected carboxyl, hydroxy, amino or thiol by a protecting group, and the other symbols are as hereinbefore defined; to deprotection.

Methyl, ethyl, t-butyl and benzyl may be used as protecting groups for carboxyl.

Methoxymethyl, 2-tetrahydropyranyl, t-butyldimethylsilyl, t-butyldiphenylsilyl, acetyl and benzyl may be used as protecting groups for hydroxy.

Benzyloxycarbonyl, t-butoxycarbonyl, trifluoroacetyl and 9-fluorenylmethoxycarbonyl may be used as protecting groups for amino.

Benzyl, methoxybenzyl, methoxymethyl, 2-tetrahydropyranyl, diphenylmethyl and acetyl may be used as protecting groups for thiol.

As protecting groups for carboxyl, hydroxy, amino or thiol, other groups, which can be removed easily and selectively other than the above protecting groups, are also preferred. For example, the groups described in T.W. Greene, Protective Groups in Organic Synthesis, Wiley, New York, 1991, may be used.

The method of deprotection for protected carboxyl, hydroxy, amino or thiol by a protecting group is known. It includes the method of

(1) deprotection under alkaline conditions,
(2) deprotection under acidic conditions,
(3) hydrogenolysis,
(4) deprotection of silyl.

These methods are explained as follows.

(1) Deprotection under alkaline conditions may be carried out, for example, in an organic solvent (e.g. methanol, tetrahydrofuran, dioxane or dimethylformamide), using an alkali metal hydroxide (e.g. sodium hydroxide, potassium hydroxide or lithium hydroxide), an alkaline earth metal hydroxide (e.g. barium hydroxide or calcium hydroxide) or a carbonate (e.g. sodium carbonate or potassium carbonate), and using an organic amine (e.g. triethylamine, diisopropylethylamine, piperadine) or quaternary ammonium (e.g. tetrabutylammonium fluoride) or an aqueous solution thereof or a mixture thereof at 0-40°C.
(2) Deprotection under acidic conditions may be carried out, for example, in a organic solvent (e.g. methylene chloride, chloroform, dioxane, ethyl acetate, anisole), using an organic acid (e.g. acetic acid, trifluoroacetic acid or methanesulfonic acid), or an inorganic acid (e.g. hydrogen chloride or sulfuric acid) or a mixture thereof (e.g. hydrogen bromide / acetic acid) at 0-100°C.
(3) Hydrogenolysis may be carried out, for example, in a solvent (ether, e.g. tetrahydrofuran, dioxane, dimethoxyethane, diethyl ether; alcohol, e.g. methanol, ethanol; benzene, e.g. benzene, toluene; ketone, e.g. acetone, methyl ethyl ketone; nitrile, e.g. acetonitrile; amide, e.g. dimethylformamide; water, ethyl acetate, acetic acid or two more mixture thereof), in the presence of a catalyst (e.g. palladium on carbon, palladium black, palladium hydroxide, platinum dioxide or Raney-nickel), at ordinary or elevated pressure of hydrogen gas or ammonium formate at 0-200°C.

(4) Deprotection of silyl may be carried out, for example, in water-miscible organic solvent (e.g. tetrahydrofuran, acetonitrile), using tetrabutylammonium fluoride at 0-40°C.

As will be apparent to those skilled in the art, the desired compound of the present invention may be easily prepared using a corresponding method selected from these methods of deprotection.

The compounds of formula (II) and formula (III) as a starting material may be prepared by methods described in scheme 1 and scheme 2 or by known methods, or they are commercially available. For example, they may be prepared by methods described in specification of the present invention.

## Scheme 1

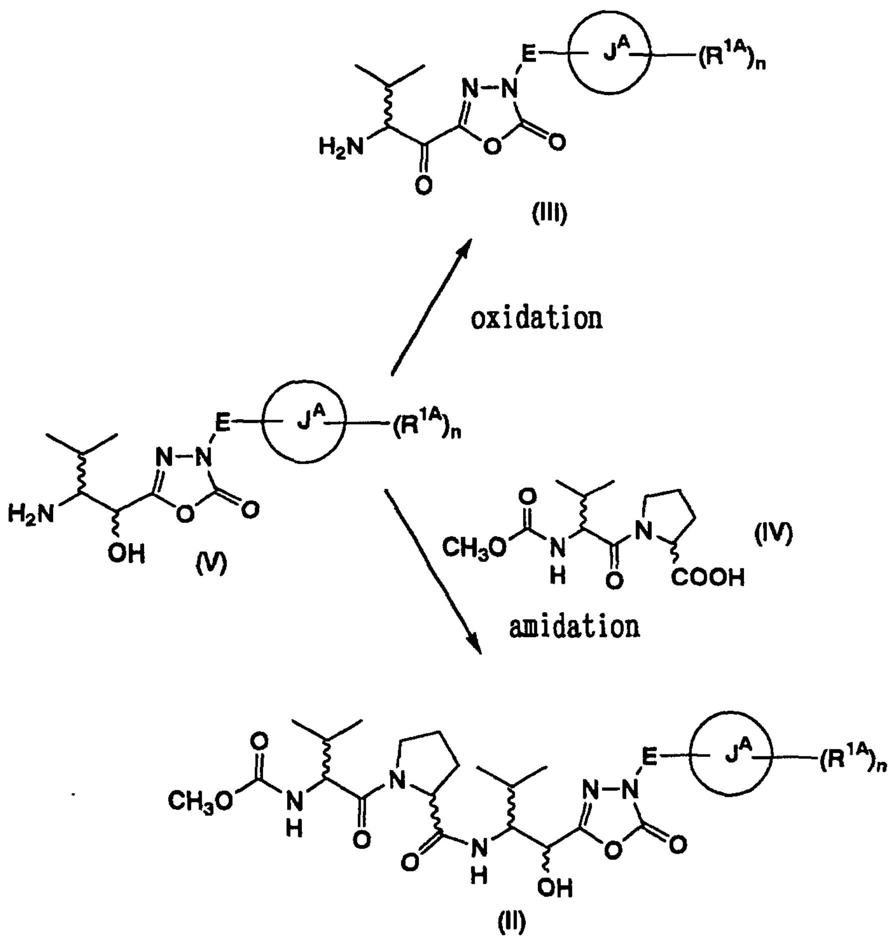

Scheme 2

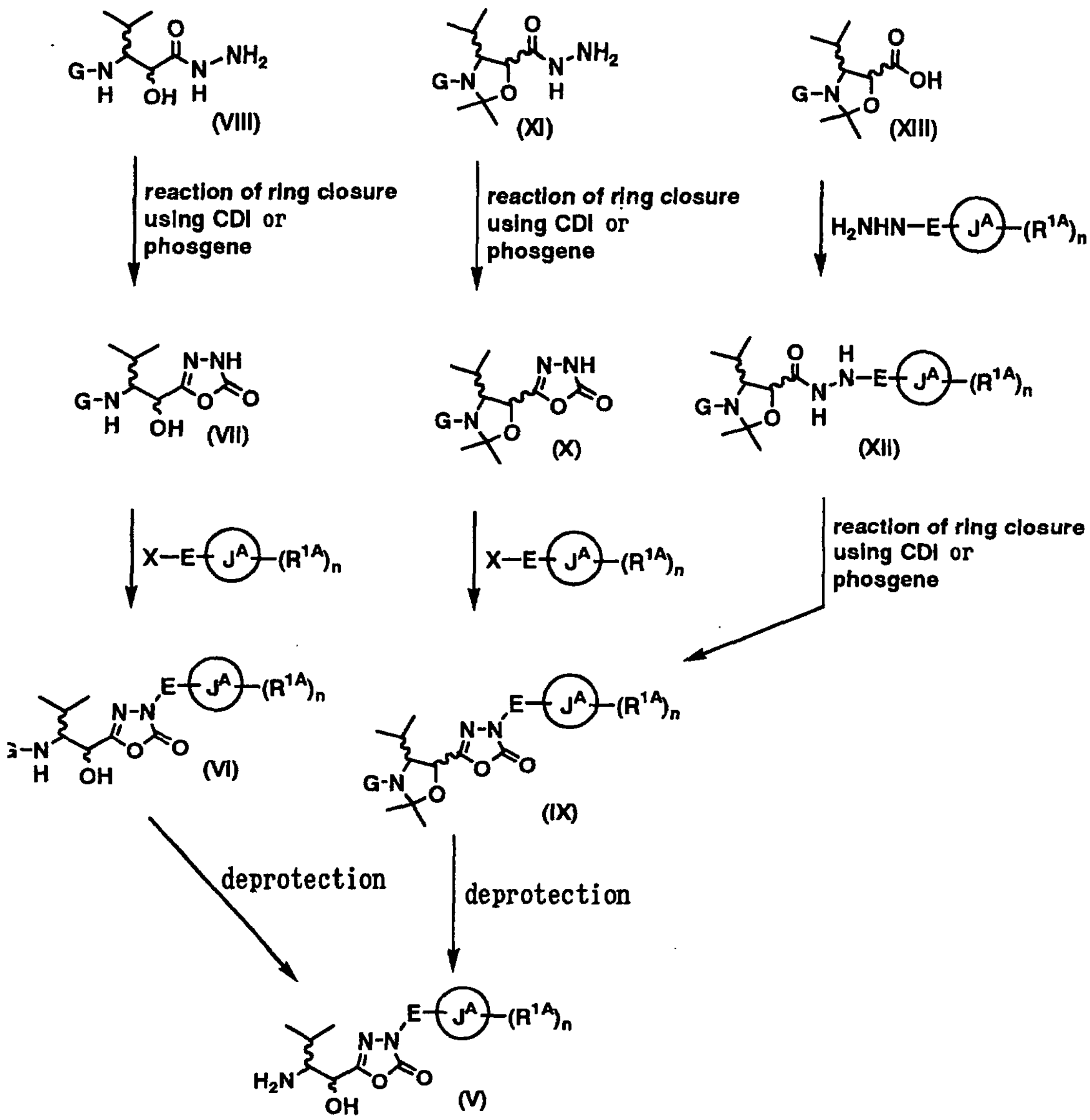


In the schemes,

G is benzyloxycarbonyl or t-butoxycarbonyl,
CDI is 1, 1'-carbonyldiimidazole,
X is halogen atom and
the other symbols are as hereinbefore defined.

Each reaction in the above schemes may be carried out by a known method. In the schemes, the compound of formula (VIII), formula (XI) or formula (XIII) as a starting material are known or may be prepared by known methods. For example, they may be prepared by methods described in specification of the present invention.

In each reaction in the present specification, products may be purified by conventional techniques. For example, purification may be carried out by distillation at atmospheric or reduced pressure, by high performance liquid chromatography, by thin layer chromatography or by column chromatography using silica gel or magnesium silicate, by washing or by recrystallization. Purification may be carried out after each reaction, or after a series of reactions.

Effect

It has been confirmed that the compounds of the present invention have an inhibitory activity against elastase by following screening tests in the laboratory.

(1) Inhibitory effects on human neutrophil elastase (HNE)

Various concentrations of test compounds or DMSO as a vehicle were added to 0.1 M HEPES buffer (pH 8.0) containing the synthetic substrate MeO-Suc-Ala-Ala-Pro-Val-p-nitroanilide (final concentration, 0.1, 0.2 or 0.4mM), 0.5 M sodium chloride solution and 0.1% Polyethyleneglycol 6000 in a final volume of 180 μL. This mixture was pre-incubated at 37°C for 5 minutes. Then, the reaction was started by the addition of 20 μL of each enzyme solution (final concentration, 0.2U/mL). The hydrolysis rate of substrate (mO.D./minute) was measured by continuously monitoring absorbance at 405 nm for 10 minutes at 30 seconds intervals. The $K_i$ values were determined by Dixon plot.

As a result, Ki value of the compound of example 1 and example 2 was 4.27 and 3.41nM, respectively.

(2) Inhibitory effect on HNE-induced lung hemorrhage in hamsters

Test compound or vehicle solution (Polyethyleneglycol 400 : ethanol : distilled water = 51 : 16 : 33) was administered orally to male Syrian hamsters. One hour after administration, animals were anesthetized with sodium pentobarbital (60 mg/kg, i.p.) and their tracheae were exposed. Saline (100 μL/lung) or HNE (10 U/100 μL/lung) were instilled intratracheally. One hour after the instillation of HNE, animals were exsanguinated by cutting the abdominal aorta. The trachea was re-exposed and cannulated, then bronchoalveolar lavage fluid (BALF) was collected by infusing and withdrawing of saline (2.5 mL/animal). Erythrocytes in 0.2 mL of BALF sample were hemolyzed by adding 1.8 mL of distilled water. The lysate was centrifuged (3000 r.p.m., 4°C, 10 minutes), and absorbance of the supernatant at 412 nm was measured spectrophotometrically to calculate blood volume in BALF using a standard curve.

As a result, $ED_{50}$ value of the compound of example 2 was 18.6mg/kg.

(3) Inhibitory effects on the increase of endogenous neutrophil elastase activity in hamster whole blood stimulated by opsonized zymosan (OZ)

Test compounds or vehicle solution (Polyethyleneglycol 400 : ethanol : distilled water = 51 : 16 : 33, or other corresponding solvents) was administered orally to male Syrian hamsters. One hour after administration, animals were anesthetized with ether, and 0.9 mL of blood samples were collected from the abdominal aorta into syringes containing a 0.1 mL of 3.8% sodium citrate. Blood samples (540 μL) were pre-incubated for 5 minutes. Then, they were incubated with 60 μL of OZ or Hank's buffer as a vehicle of OZ at 37°C. Thirty minutes later, the reaction was stopped by cooling on ice-water. Samples were centrifuged (3000 r.p.m., 4°C, 10 minutes). NE activity in plasma was determined using the synthetic substrate MeO-Suc-Ala-Ala-Pro-Val-p-nitroanilide. Plasma samples were incubated in 0.1 M Tris-HCl buffer (pH 8.0) containing MeO-Suc-Ala-Ala-Pro-Val-p-nitroanilide (final concentration, 1 mM) and 0.5 M sodium chloride solution in a final volume of 200 μL at 37°C for 24 hours, and absorbance at 405 nm was measured spectrophotometrically. The amount of p-nitroaniline released from substrate was regarded as NE activity. Percent inhibition of a compound was calculated by the following equation.

$$\text{Inhibition (\%)} = [1 - \{\text{deltaOD (test}-\text{blank) / deltaOD (control}-\text{blank)}\}] \times 100$$

As a result, it has confirmed that the compounds of the present invention have an inhibitory activity against elastase, especially, have an inhibitory activity against elastase when orally administered.

Toxicity

The toxicity of the compounds of the present invention is very low and therefore the compounds may be considered safe for pharmaceutical use.

Industrial Applicability

Application for Pharmaceuticals

The compounds of the present invention of formula (I), a non-toxic salt thereof and an acidic addition salt

have an inhibitory activity on elastase and are useful for treatment and / or prevention of a disease induced by an abnormal enhancement of degradation of elastin, collagen fiber and / or proteoglycans by elastase, for example, pulmonary emphysema, chronic obstructive pulmonary disease, rheumatoid arthritis, arteriosclerosis, adult respiratory distress syndrome (ARDS), glomerular nephritis, myocardial infarction, ulcerative colitis and gingivitis.

For the purpose above described, the compounds of formulae (I) of the present invention, a non-toxic salt thereof or hydrates may be normally by administered systemically or locally, usually by oral or parenteral administration.

The doses to be administered are determined depending upon, for example, age, body weight, symptom, the desired therapeutic effect, the route of administration, and the duration of the treatment. In the human adult, the doses per person are generally from 1 mg to 1000 mg, by oral administration, up to several times per day, and from 1 mg to 100 mg, by parenteral administration (preferably intravenous administration), up to several times per day, or continuous administration from 1 to 24 hours per day from vein.

As mentioned above, the doses to be used depend upon various conditions. Therefore, there are cases in which doses lower than or greater than the ranges specified above may be used.

The compound of formula (I) may be administered in the form of, for example, solid compositions, liquid compositions or other compositions for oral administration, injections, liniments or suppositories for parenteral administration.

Solid compositions for oral administration include compressed tablets, pills, capsules, dispersible powders, and granules.

Capsules include hard capsules and soft capsules.

In such compositions, one or more of the active compound(s) may be admixed with at least one inert diluent (such as lactose, mannitol, glucose, hydroxypropyl cellulose, microcrystalline cellulose, starch, polyvinylpyrrolidone or magnesium metasilicate aluminate). The compositions may also comprise, as is normal practice, additional substances other than inert diluents: e.g. lubricating agents (such as magnesium stearate), disintegrating agents (such as cellulose calcium glycolate), stabilizing agents, and agents to assist dissolution (such as glutamic acid or aspartic acid).

The tablets or pills may, if desired, be coated with a film of gastric or enteric material (such as sugar, gelatin, hydroxypropyl cellulose or hydroxypropylmethyl cellulose phthalate), or be coated with two or more films. And further, coating may include containment within capsules of absorbable materials such as gelatin.

Liquid compositions for oral administration include pharmaceutically acceptable emulsions, solutions, syrups and elixirs. In such compositions, one or more of the active compound(s) may be contained in an inert diluent(s) commonly used in the art (e.g. purified water or ethanol). Besides inert diluents, such compositions may also comprise adjuvants (such as wetting agents or suspending agents), sweetening agents, flavoring agents, perfuming agents, and preserving agents.

Other compositions for oral administration include spray compositions which may be prepared by known methods and which comprise one or more of the active compound(s). Spray compositions may comprise additional substances other than inert diluents: e.g. stabilizing agents (such as sodium sulfate), isotonic buffer (such as sodium chloride, sodium citrate or citric acid). For preparation of such spray compositions, for example, the method described in the United States Patent No. 2,868,691 or 3,095,355 may be used.

Injections for parenteral administration include sterile aqueous or non-aqueous solutions, suspensions and emulsions. Aqueous solutions and suspensions may include distilled water for injection or physiological salt solution. Non-aqueous solutions and suspensions may include propylene glycol, polyethylene glycol, vegetable oil such as olive oil, alcohol such as ethanol or POLYSORBATE80 (registered trade mark).

Injections may comprise additional ingredients other than inert diluents: e.g. preserving agents, wetting agents, emulsifying agents, dispersing agents, stabilizing agents, assisting agents such as agents to assist dissolution (e.g. glutamic acid or aspartic acid).

They may be sterilized for example, by filtration through a bacteria-retaining filter, by incorporation of sterilizing agents in the compositions or by irradiation. They may also be manufactured in the form of sterile solid compositions, for example, freeze-dried products, which may be dissolved in sterile water or some other sterile diluent(s) for injection immediately before use.

Other compositions for parenteral administration include liquids for external use, and endermic liniments, ointment, suppositories for rectal administration and pessaries for vaginal administration which comprise one or more of the active compound(s) and may be prepared by methods known per se.

Best mode for carrying out the invention

The following reference examples and examples illustrate, but do not limit the present invention.

The solvents in parenthesis show the developing or eluting solvents and the ratios of the solvents used are by volume in chromatographic separations and TLC.

The NMR data are shown with the solvent used in the measurements, in parentheses.

Reference example 1

N-[1-((2R)-oxiran-2-yl)-(1S)-2-methylpropyl] (t-butoxy)carboxamide

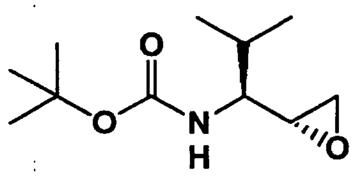

To a solution of 60% sodium hydride (8.9 g) in dimethylformamide (250 ml), under an argon atmosphere, trimethylsulfonium iodide (52.4 g) was added at room temperature. After the mixture was stirred for 1 hour, a solution of N-(t-butoxycarbonyl)valinal (34 g) in tetrahydrofuran (75 ml) was dropped into the mixture at room temperature. The mixture was stirred for 45 minutes at room temperature. The reaction mixture was poured into ice water and the solution was extracted with ethyl acetate. The extract was washed with water and a saturated aqueous solution of sodium chloride, dried over anhydrous sodium sulfate and concentrated. The residue was purified by column chromatography on silica gel (hexane : ethyl acetate = 19 : 1 → 9 : 1 → 5 : 1) to give the title compound (18.4 g) having the following physical data.
TLC: Rf 0.55 (hexane : acetyl acetate = 2 : 1);
NMR ($CDCl_3$): δ 4.54-4.40 (m, 1H), 3.78-3.68 (m, 1H), 3.07 (m, 1H), 2.69 (t, J = 4.5 Hz, 1H), 2.53 (m, 1H), 1.98-1.85 (m, 1H), 1.43 (s, 9H), 1.02 and 0.99 (each d, J = 6.9 Hz, each 3H).

Reference example 2

N-(t-butoxycarbonyl)-(1S)-(2-propyl)-(2R)-hydroxy-3-acetyloxypropylamine

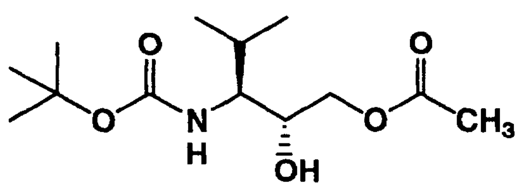

To a solution of the compound prepared in reference example 1 (15.7 g) in dimethylformamide (320 ml), acetic acid (6.26 ml) and lithium carbonate (27 g) were added. The mixture was stirred for 4 days at 100°C. The reaction mixture was poured into 10% aqueous solution of citric acid, and the solution was extracted with ethyl acetate. The extract was washed with 10% aqueous solution of citric acid, a saturated aqueous solution of sodium bicarbonate and a saturated aqueous solution of sodium chloride, dried over anhydrous sodium sulfate and concentrated. The residue was purified by column chromatography on silica gel (hexane : ethyl acetate = 4 : 1 → 2 : 1 → 1 : 1) to give the title compound (9.66 g) having the following physical data.
TLC: Rf 0.32 (hexane : acetyl acetate = 2 : 1);
NMR ($CDCl_3$): δ 4.82 (d, J = 9.3 Hz, 1H), 4.19-3.96 (m, 3H), 3.31-3.20 (m, 1H), 2.76 (d, J = 4.2 Hz, 1H), 2.10 (s, 3H), 2.02-1.80 (m, 1H), 1.44 (s, 9H), 0.98 and 0.96 (each d, J = 6.9 Hz, each 3H).

Reference example 3

N-(t-butoxycarbonyl)-2, 2-dimethyl-(4S)-(2-propyl)-(5R)-acetyloxymethyloxazolidine

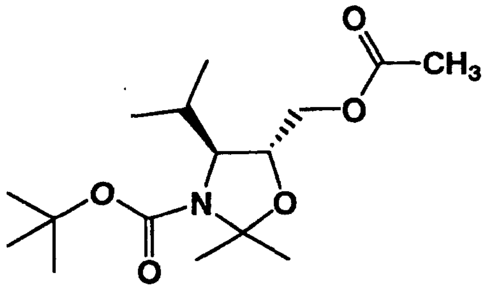

To a solution of the compound prepared in reference example 2 (9.66 g) in dimethylformamide (40 ml), isopropenyl methyl ether (10.1 ml) and camphorsulfonic acid (405 mg) were added. The mixture was stirred for 2 hours at room temperature. The reaction mixture was poured into a saturated aqueous solution of sodium bicarbonate, and the solution was extracted with ethyl acetate. The extract was washed with a saturated aqueous solution of sodium chloride, dried over anhydrous sodium sulfate and concentrated. The residue was purified by column chromatography on silica gel (hexane : ethyl acetate = 40 : 1 → 19 : 1 → 10 : 1 → 2 : 1) to give the title compound (10 g) having the following physical data.
TLC: Rf 0.50 (hexane : acetyl acetate = 4 : 1);
NMR ($CDCl_3$): δ 4.20-4.02 (m, 3H), 3.80-3.40 (m, 1H), 2.34-2.12 (m, 1H), 2.10 (s, 3H), 1.59 and 1.54 (each s, each 3H), 1.48 (s, 9H), 0.92 and 0.91 (each d, J = 6.9 Hz, each 3H).

Reference example 4

N-(t-butoxycarbonyl)-2, 2-dimethyl-(4S)-(2-propyl)-(5R)-hydroxymethyloxazolidine

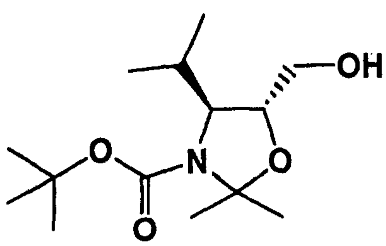

To a solution of the compound prepared in reference example 3 (10 g) in methanol (50 ml), potassium carbonate (4.83 g) was added. The mixture was stirred for 3 hours at room temperature. The reaction mixture was poured into ice water, and the solution was extracted with ethyl acetate. The extract was washed with water and a saturated aqueous solution of sodium chloride, dried over anhydrous sodium sulfate and concentrated to give the title compound (8.74 g) having the following physical data.
TLC: Rf 0.21 (hexane : acetyl acetate = 4 : 1);
NMR ($CDCl_3$): δ 4.07-4.00 (m, 1H), 3.66-3.56 (m, 3H), 2.38-2.20 (m, 1H), 1.93 (m, 1H), 1.61 and 1.54 (each s, each 3H), 1.47 (s, 9H), 0.91 and 0.90 (each d, J = 6.9 Hz, each 3H).

Reference example 5

N-(t-butoxycarbonyl)-2, 2-dimethyl-(4S)-(2-propyl)-(5R)-carboxyoxazolidine

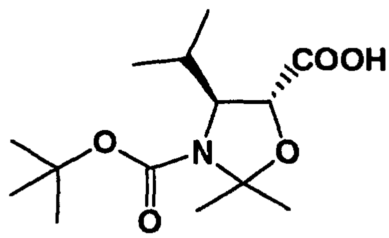

To a solution of the compound prepared in reference example 4 (8.68 g) in carbon tetrachloride (85 ml), acetonitrile (85 ml) and water (128 ml), sodium metaperiodate (20.4 g) and ruthenium chloride hydrate (87 mg, 1%) were added. The mixture was stirred overnight at room temperature. The reaction mixture was filtered through Celite (registered trademark), and was washed with dichloromethane. The filtrate was washed with water, dried over anhydrous sodium sulfate and concentrated. The residue was purified by column chromatography on silica gel (chloroform : methanol = 100 : 1 → 10 : 1 and hexane : ethyl acetate = 2 : 1) to give the title compound (5.23 g) having the following physical data.
TLC: Rf 0.33 (chloroform : methanol = 9 : 1);
NMR ($CDCl_3$): δ 4.38 (d, J = 2.7 H, 1H,), 4.27-4.17 (m, 1H,), 2.36-2.17 (m, 1H), 1.63 and 1.61 (each s, each 3H), 1.47 (s, 9H), 0.97 and 0.95 (each d, J = 6.9 Hz, each 3H).

Reference example 6

N-(t-butoxycarbonyl)-2, 2-dimethyl-(4S)-(2-propyl)-(5R)-methoxycarbonyloxazolidine

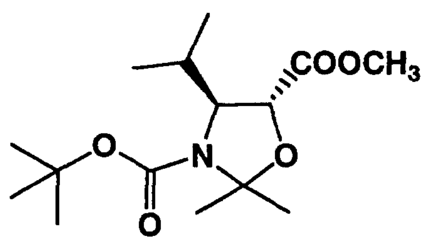

To a solution of the compound prepared in reference example 5 (16 g) in ethyl acetate (100 ml), a solution of diazomethane in diethyl ether (90 ml) was added at 0°C. The mixture was concentrated to give the title compound (17.5 g) having the following physical data.
TLC: Rf 0.32 (hexane : ethyl acetate = 9 : 1);
NMR ($CDCl_3$): δ 4.36 (d, J = 2.4 Hz, 1H), 4.28-4.05 (m, 1H), 3.78 (s, 3H), 2.38-2.10 (m, 1H), 1.58 (s, 6H), 1.47 (s, 9H), 0.95 and 0.93 (each d, J = 6.9 Hz, each 3H).

Reference example 7

[N-(t-butoxycarbonyl)-2, 2-dimethyl-(4S)-(2-propyl)oxazolidin-(5R)-yl] carbohydrazide

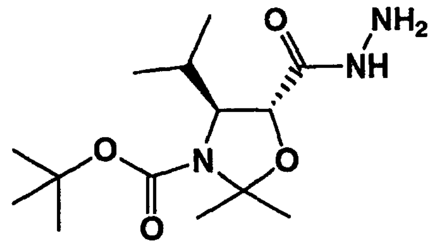

A solution of the compound prepared in reference example 6 (17.1 g) in methanol (30 ml) was added to hydrazine hydrate (55.7 g) at room temperature. Methanol (10 ml) was added to the mixture, and the mixture was stirred for 2 hours at room temperature. The reaction mixture was poured into ice water, and the solution was extracted with dichloromethane. The extract was washed with water and a saturated aqueous solution of sodium chloride, dried over anhydrous sodium sulfate and concentrated to give the title compound (17.1 g) having the following physical data.
TLC: Rf 0.51 (ethyl acetate);
NMR ($CDCl_3$): δ 7.63 (br, 1H), 4.30 (d, J = 3.0 Hz, 1H), 4.28-4.22 (m, 1H), 2.37-2.17 (m, 1H), 1.59 and 1.58 (each s, each 3H), 1.46 (s, 9H), 0.98 and 0.94 (each d, J = 6.9 Hz, each 3H).

Reference example 8

N-(t-butoxycarbonyl)-2, 2-dimethyl-(4S)-(2-propyl)- (5R)-(1,3,4-oxadiazolin-2-one-5-yl)oxazolidine

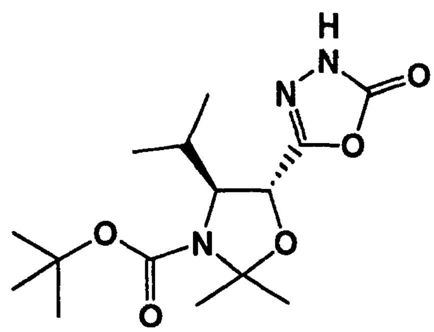

To a solution of the compound prepared in reference example 7 (16.76 g) in tetrahydrofuran (500 ml), under an argon atmosphere, triethylamine (10.07 ml) and 1, 1'-carbonyldiimidazole (10.76 g) were added at room temperature. The mixture was stirred overnight at room temperature. The reaction mixture was poured into ice water, and the solution was extracted with ethyl acetate. The extract was washed with 10% aqueous solution of citric acid and a saturated aqueous solution of sodium chloride, dried over anhydrous sodium sulfate and concentrated. The residue was purified by column chromatography on silica gel (hexane : ethyl acetate = 4 : 1) to give the title compound (16.76 g) having the following physical data.
TLC: Rf 0.19 (hexane : ethyl acetate = 4 : 1);
NMR ($CDCl_3$): δ 9.28 (br, 1H), 4.75 (d, J = 4.2 Hz, 1H), 4.50-4.15 (m, 1H), 2.46-2.15 (m, 1H), 1.59 and 1.53 (each s, each 3H), 1.49 (s, 9H), 0.96 and 0.92 (each d, J = 6.6 Hz, each 3H).

Reference example 9

N-(t-butoxycarbonyl)-2, 2-dimethyl-(4S)-(2-propyl)-(5R)-[3-thiophen-3-ylmethyl-1,3,4-oxadiazolin-2-one-5-yl] oxazolidine

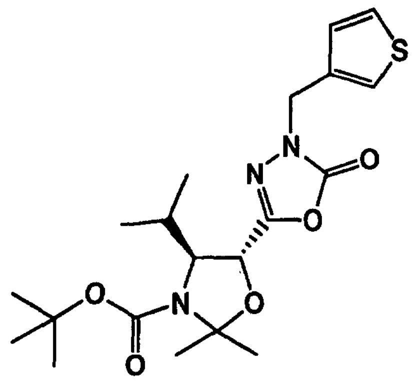

To a solution of the compound prepared in reference example 8 (0.507 g) in dimethylformamide (1.5 ml), potassium carbonate (235 mg) and 3-bromomethylthiophene (303 mg) were added. The mixture was stirred for 2 hours at room temperature. The reaction mixture was poured into 10% aqueous solution of citric acid, and the solution was extracted with ethyl acetate. The extract was washed with a saturated aqueous solution of sodium chloride, dried over anhydrous sodium sulfate and concentrated. The residue was purified by column chromatography on silica gel (hexane : ethyl acetate = 25 : 1 → 15 : 1) to give the title compound (640 mg) having the following physical data.
TLC: Rf 0.23 (hexane : ethyl acetate = 4 : 1);
NMR ($CDCl_3$): δ 7.30-7.25 (m, 2H), 7.09-7.08 (m, 1H), 4.87 (s, 2H), 4.72 (d, J = 3.0 Hz, 1H), 4.25 (brs, 1H), 2.28 (brs, 1H), 1.56 (s, 9H), 1.47 (s, 6H), 0.93 and 0.90 (each d, J = 6.6 Hz, each 3H).

Reference example 10

(1S)-[(3-(thiophen-3-ylmethyl)-1,3,4-oxadiazolin-2-one-5-yl)-(1R)-hydroxymethyl]-2-methylpropylamine p-toluenesulfonate

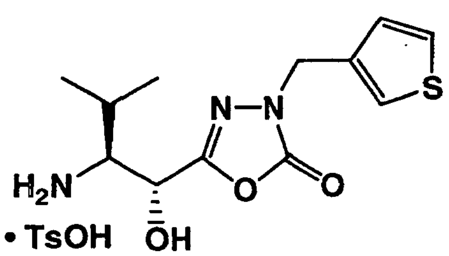

To a solution of the compound prepared in reference example 9 (430 mg) in ethanol (15 ml), p-toluenesulfonic acid (270 mg) was added. The mixture was stirred for 16 hours at 80°C. The reaction mixture was concentrated. The residue was solidified again using diethyl ether and dried to give the title compound (419 mg) having the following physical data.
TLC: Rf 0.28 (chloroform : methanol = 10 : 1);
NMR (DMSO-$d_6$): δ 7.93 (brs, 3H), 7.50-7.46 (m, 4H), 7.13-7.08 (m, 3H), 6.96 (d, J = 6.0 Hz, 1H), 4.88 (s, 2H), 4.80-4.76 (m, 1H), 3.21 (brs, 1H), 2.29 (s, 3H), 1.98-1.88 (m, 1H), 0.98 and 0.92 (each d, J = 6.8 Hz, each 3H).

Reference example 11

(methoxycarbonyl)-L-valyl-N-[(1S)-([3-(thiophen-3-ylmethyl)-1,3,4-oxadiazolin-2-one-5-yl]-(1R)-hydroxymethyl)-2-methylpropyl]-L-prolinamide

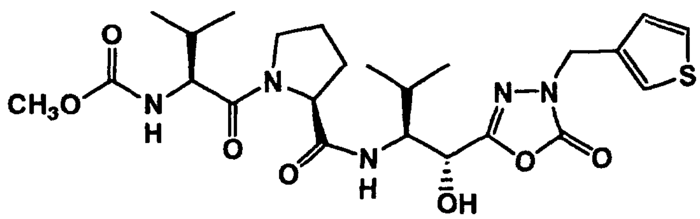

To a solution of the compound prepared in reference example 10 (0.22 g) and (methoxycarbonyl)-L-valyl-L-proline (0.131 g) in dimethylformamide (2 ml), under an argon atmosphere, 1-hydroxybenzotriazole hydrate (87 mg), 1-ethyl-3-[3-(dimehtylamino)propyl]carbodiimide hydrochloride (101 mg) and 4-methylmorpholine (90 µl) were added at 0°C. The mixture was stirred overnight at room temperature. The reaction mixture was poured into 10% aqueous solution of citric acid, and the solution was extracted with ethyl acetate. The extract was washed with a saturated aqueous solution sodium bicarbonate and a saturated aqueous solution sodium chloride, dried over anhydrous sodium sulfate and concentrated. The residue was purified by column chromatography on silica gel (chloroform : methanol = 100 : 1) to give the title compound (219 mg) having the following physical data.
TLC: Rf 0.40 (chloroform : methanol = 10 : 1);
NMR (DMSO-$d_6$): δ 7.76 (d, J = 9.4 Hz, 1H), 7.53 (dd, J = 4.4, 3.0 Hz, 1H), 7.46-7.44 (m, 1H), 7.22 (d, J = 8.8 Hz, 1H), 7.06-7.02 (m, 1H), 5.99 (d, J = 5.6 Hz, 1H), 4.86 (s, 2H), 4.68-4.64 (m, 1H), 4.38-4.30 (m, 1H), 3.97 (t, J = 8.4 Hz, 1H), 3.74-3.60 (m, 2H), 3.55-3.47 (m, 4H), 1.93-1.61 (m, 6H), 0.97-0.84 (m, 12H).

Example 1

(methoxycarbonyl)-L-valyl-N-[(1S)-([3-(thiophen-3-ylmethyl)-1,3,4-oxadiazolin-2-one-5-yl]carbonyl)-2-methylpropyl] -L-prolinamide

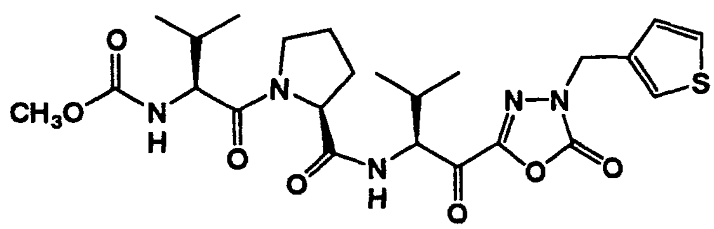

To a solution of oxalyl chloride (48 µl) in dichloromethane (2.6 ml), under an argon atmosphere, a solution of 1M dimethylsulfoxide in dichloromethane (1.1 ml) was dropped slowly at -78°C. The mixture was stirred for 30 minutes at -78°C. A solution of the compound prepared in reference example 11 (148 mg) in dichloromethane (1.5 ml) was dropped to the mixture at -78°C. After the mixture was stirred for 1.5 hours at same temperature. 4-Methylmorpholine (0.24 ml) was added to the reaction mixture at -78°C. The mixture was stirred for 30 minutes at -20°C. 1 mol/$m^3$ Hydrochloric acid was added to the reaction mixture, and the solution was extracted with ethyl acetate. The extract was washed with a saturated aqueous solution of sodium chloride, dried over anhydrous sodium sulfate and concentrated. The residue was purified by column chromatography on silica gel (chloroform : methanol = 100 : 1) to give the title compound (120 mg) having the following physical data.
TLC: Rf 0.13 (hexane : ethyl acetate = 1 : 1);
NMR (DMSO-$d_6$): δ 8.37 (d, J = 6.9 Hz, 1H), 7.57-7.54 (m, 2H), 7.26 (d, J = 8.4 Hz, 1H), 7.12-7.10 (m, 1H), 5.01 (s, 2H), 4.81 (t, J = 6.9 Hz, 1H), 4.43 (dd, J = 8.4, 4.2 Hz, 1H), 3.98 (t, J = 8.4 Hz, 1H), 3.76-3.67 and 3.55-3.45 (each m, totally 5H), 2.26-2.14 and 2.02-1.66 (each m, totally 6H), 0.92-0.84 (m, 12H).

Example 1(1)-(5)

The following compounds of the present invention were obtained by the same procedure as a series of reaction of Reference Example 10 → Reference Example 11 → Example 1, using a corresponding halide in stead of 3-bromomethylthiophene.

Example 1(1)

(methoxycarbonyl)-L-valyl-N-[(1S)-([3-benzyl-1,3,4-oxadiazolin-2-one-5-yl]carbonyl)-2-methylpropyl]-L-prolinamide

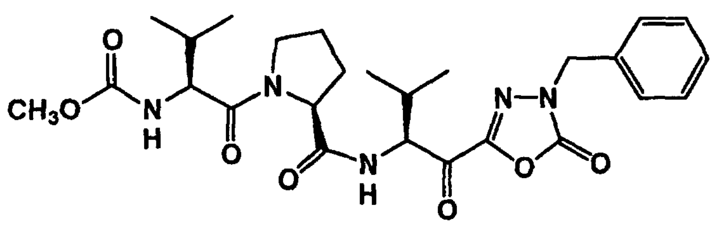

TLC: Rf 0.46 (chloroform : methanol = 19 : 1);
NMR ($CDCl_3$): δ 7.47-7.30 (m, 1H), 7.37 (m, 5H), 5.39 (d, J = 9.2 Hz, 1H), 5.10 (dd, J = 7.2 Hz, 5.4 Hz, 1H), 4.97 (s, 2H), 4.62-4.52 (m, 1H), 4.30 (dd, J = 9.2 Hz, 7.0 Hz, 1H), 3.83-3.47 (m, 2H), 3.67 (s, 3H), 2.37-1.75 (m, 6H), 1.07-0.84 (m, 12H).

Example 1(2)

(methoxycarbonyl)-L-valyl-N-[(IS)-([3-(2-phenylethyl)-1,3,4-oxadiazolin-2-one-5-yl]carbonyl)-2-methylpropyl]-L-prolinamide

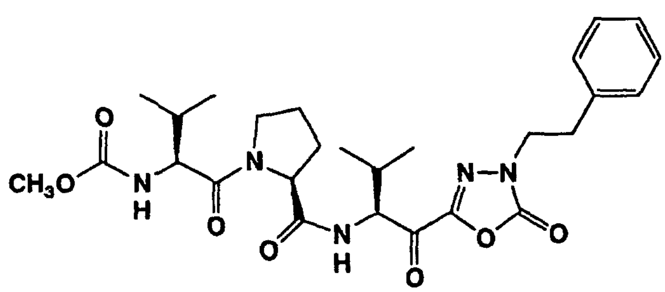

TLC: Rf 0.58 (ethyl acetate);
NMR ($CDCl_3$): δ 7.39 (d, J = 7.2 Hz, 1H), 7.38-7.09 (m, 5H), 5.35 (d, J = 8.9 Hz, 1H), 5.09 (dd, J = 7.2 and 5.4 Hz, 1H), 4.60 (dd, J = 8.1 and 3.0 Hz, 1H), 4.31 (dd, J = 8.9 and 7.2 Hz, 1H), 4.23-3.96 (m, 2H), 3.86-3.47 (m, 2H), 3.68 (s, 3H), 3.11 (t, J = 7.2 Hz, 2H), 2.41-2.23 (m, 1H), 2.23-1.78 (m, 5H), 1.12-0.75 (m, 12H).

Example 1(3)

(methoxycarbonyl)-L-valyl-N-[(1S)-([3-(3,4-methylenedioxyphenylmethyl)-1,3,4-oxadiazolin-2-one-5-yl]carbonyl)-2-methylpropyl]-L-prolinamide

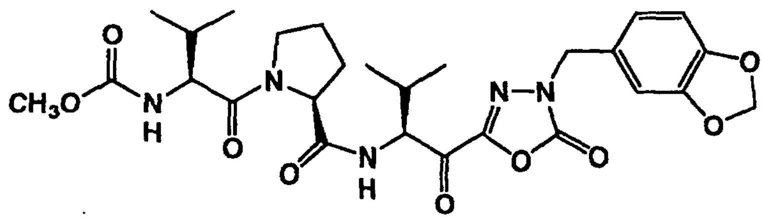

TLC: Rf 0.63 (ethyl acetate);
NMR ($CDCl_3$): δ 7.39 (d, J = 7.5 Hz, 1H), 6.90-6.83 (m, 2H), 6.78 (d, J = 8.7 Hz, 1H), 5.97 (s, 2H), 5.36 (d, J = 9.2 Hz, 1H), 5.10 (dd, J = 7.5 and 5.4 Hz, 1H), 4.86 (s, 2H), 4.58 (dd, J = 7.5 and 2.7 Hz, 1H), 4.31 (dd, J = 9.2 and 6.9 Hz, 1H), 3.84-3.50 (m, 2H), 3.67 (s, 3H), 2.40-1.80 (m, 6H), 1.08-0.80 (m, 12H).

Example 1(4)

(methoxycarbonyl)-L-valyl-N-[(1S)-([3-(thiophen-2-ylmethyl)-1,3,4-oxadiazolin-2-one-5-yl]carbonyl)-2-methylpropyl] -L-prolmamide

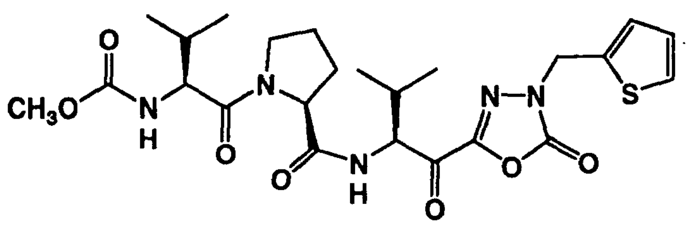

TLC: Rf 0.34 (chloroform : methanol = 20 : 1);
NMR (DMSO-$d_6$): δ 8.39 (d, J = 7.0 Hz, 1H), 7.54 (dd, J = 5.0, 1.0 Hz, 1H), 7.27 (d, J = 8.5 Hz, 1H), 7.18 (d, J = 3.5 Hz, 1H), 7.02 (dd, J = 5.0, 3.5 Hz, 1H), 5.21 (s, 2H), 4.80 (t, J = 7.0 Hz, 1H), 4.44 (dd, J = 7.5, 4.0 Hz, 1H), 3.97 (t, J = 8.5 Hz, 1H), 3.75-3.67 and 3.58-3.46 (each m, totally 5H), 2.25-2.14 and 2.02-1.65 (each m, totally 6H), 0.92-0.85 (m, 12H).

Example 1(5)

(methoxycarbonyl)-L-valyl-N-[(1S)-([3-(4-fluorophenylmethyl)-1,3,4-oxadiazolin-2-one-5-yl]carbonyl)-2-methylpropyl] -L-prolinamide

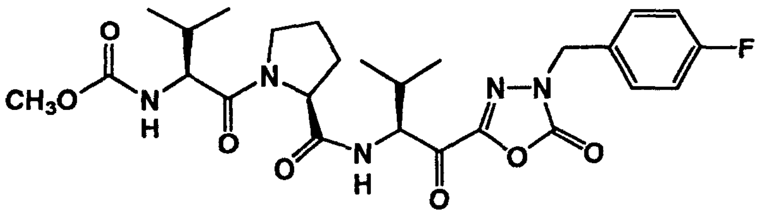

TLC: Rf 0.56 (chloroform : methanol = 19 : 1);
NMR ($CDCl_3$): δ 7.44-7.38 (m, 1H), 7.37 (dd, J = 8.4, 4.8 Hz, 2H), 7.06 (t, J = 8.4 Hz, 2H), 5.37 (d, J = 9.0 Hz, 1H), 5.09 (dd, J = 7.2, 5.7 Hz, 1H), 4.96 and 4.91 (each d, J = 15.9 Hz, each 1H), 4.57 (dd, J = 8.1, 3.0 Hz, 1H), 4.30 (dd, J = 9.0, 6.9 Hz, 1H), 3.73-3.54 (m, 2H), 3.67 (s, 3H), 2.34-1.81 (m, 6H), 1.00, 0.97, 0.96 and 0.89 (each d, J = 6.9 Hz, each 3H).

Reference example 12

N-(t-butoxycarbonyl)-2,2-dimethyl-(4S)-(2-propyl)-(5R)-[N-(phenylamino)carbamoyl]-1,3-oxazolidine

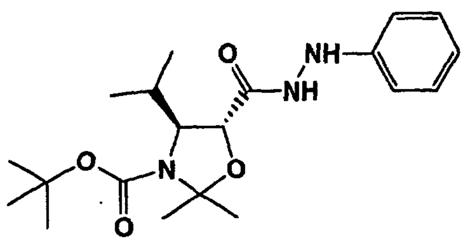

The title compound having the following physical data was obtained by the same procedure as Reference example 7, using phenylhydrazine in stead of hydrazine hydrate.
TLC: Rf 0.15 (hexane : ethyl acetate = 4 : 1);
NMR ($CDCl_3$): δ 8.26-8.13 (m, 1H), 7.32-7.17 and 6.98-6.80 (m, total 5H), 6.04 (d, J = 4.8 Hz, 1H), 4.39 (d, J = 3.2 Hz, 1H), 4.34-4.22 (m, 1H), 2.43-2.17 (m, 1H), 1.70 and 1.64 (each s, each 3H), 1.47 (s, 9H), 0.96 and 0.94 (each d, J = 6.8 Hz, each 3H).

Reference example 13

N-(t-butoxycarbonyl)-2,2-dimethyl-(4S)-(2-propyl)-(5R)-(3-phenyl-1,3,4-oxadiazolin-2-one-5-yl)oxazolidine

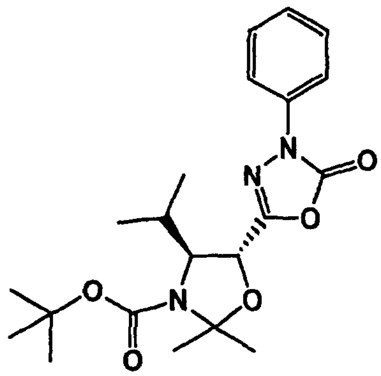

The title compound having the following physical data was obtained by the same procedure as Reference example 8, using the compound prepared in Reference example 12 in stead of the compound prepared in Reference example 7.
TLC: Rf 0.64 (hexane : ethyl acetate = 4 : 1);
NMR ($CDCl_3$): δ 7.88-7.80, 7.51-7.38 and 7.32-7.22 (each m, total 5H), 4.85 (d, J = 4.5 Hz, 1H), 4.52-4.22 (m, 1H), 2.50-2.10 (m, 1H), 1.62 and 1.60 (each s, each 3H), 1.50 (s, 9H), 1.00 and 0.96 (each d, J = 6.3 Hz, each 3H).

Reference example 14

(1S)-[(3-phenyl-1,3,4-oxadiazolin-2-one-5-yl)-(1R)-hydroxymethyl]-2-methylpropylamine p-toluenesulfonate

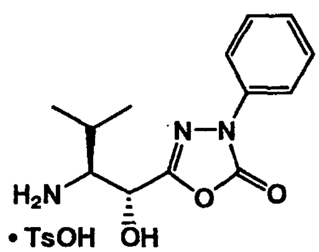

The title compound having the following physical data was obtained by the same procedure as Reference example 9, using the compound prepared in Reference example 13 in stead of the compound prepared in Reference example 8.
TLC: Rf 0.30 (chloroform : methanol = 9 : 1);
NMR (DMSO-$d_6$): δ 8.08-7.88 (br, 3H), 7.80-7.74 (m, 2H), 7.58-7.50 (m, 2H), 7.48 (d, J = 7.8 Hz, 2H), 7.38-7.30 (m, 1H), 7.15-7.06 (m, 1H), 7.11 (d, J = 7.8 Hz, 2H), 4.95 (m, 1H), 3.35-3.25 (m, 1H), 2.29 (s, 3H), 2.11-1.98 (m, 1H), 1.04 and 1.01 (each d, J = 6.9 Hz, each 3H).

Reference example 15

(methoxycarbonyl)-L-valyl-N-[(1S)-([3-phenyl-1,3,4-oxadiazolin-2-one-5-yl]-(1R)-hydroxymethyl)-2-methylpropyl]-L-prolinamide

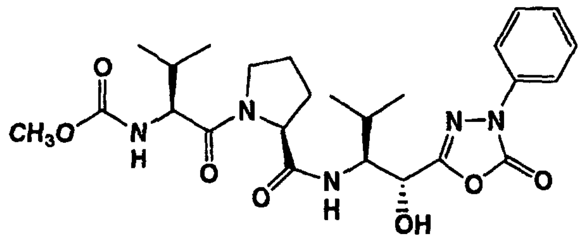

The title compound having the following physical data was obtained by the same procedure as Reference example 11, using the compound prepared in Reference example 14 in stead of the compound prepared in Reference example 10.
TLC: Rf 0.29 (chloroform : methanol = 9 : 1);
NMR (DMSO-$d_6$): δ 7.85 (d, J = 9.3 Hz, 1H), 7.80-7.71, 7.55-7.46 and 7.34-7.25 (total 5H), 7.19 (d, J = 9.0 Hz, 1H), 6.15 (d, J = 5.7 Hz, 1H), 4.85-4.77 (m, 1H), 4.42-4.32 (m, 1H), 4.00-3.88 (m, 1H), 3.77-3.40 (m, 3H), 3.50 (s, 3H), 2.05-1.58 (m, total 6H), 0.99, 0.92, 0.86 and 0.83 (each d, J = 6.6 Hz, each 3H).

Example 2

(methoxycarbonyl)-L-valyl-N-[(1S)-([3-phenyl-1,3,4-oxadiazolin-2-one-5-yl] carbonyl)-2-methylpropyl]-L-prolinamide

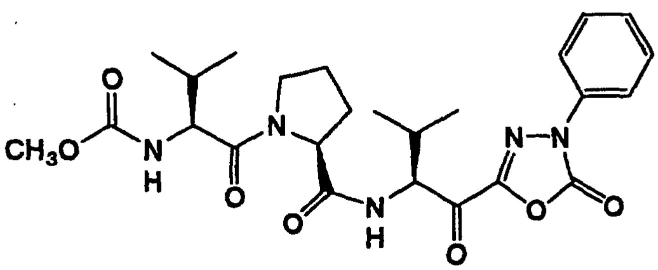

The title compound having the following physical data was obtained by the same procedure as Example 1, using the compound prepared in Reference example 15 in stead of the compound prepared in Reference example 11.
TLC: Rf 0.40 (hexane : ethyl acetate = 1 : 2);
NMR ($CDCl_3$): δ 7.89 (d, J = 8.4 Hz, 2H), 7.56-7.44 (m, 3H), 7.39-7.31 (m, 1H), 5.35 (d, J = 8.7 Hz, 1H), 5.21 (dd, J = 7.5, 5.7 Hz, 1H), 4.63 (dd, J = 7.8, 3.0 Hz, 1H), 4.32 (dd, J = 8.7, 6.9 Hz, 1H), 3.85-3.53 (m, 2H), 3.68 (s, 3H), 2.40-1.82 (m, total 6H), 1.06 and 1.01 (each d, J = 6.6 Hz, each 3H), 0.97 (d, J = 6.9 Hz, 6H).

Reference example 16

N-(t-butoxycarbonyl)-2,2-dimethyl-(4S)-(2-propyl)-(5R)-[N-((4-methoxy-2-methylphenyl)amino)carbamoyl]-1,3-oxazolidine

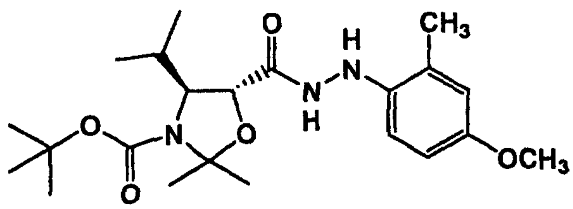

To a solution of the compound prepared in Reference example 5 (678 mg) in dimethylformamide (5 ml), under an argon atmosphere, 4-methoxy-2-methylphenyl hydrazine (445 mg), 1-hydroxybenzotriazole hydrate (433 mg) and 1-ehyl-3-[3-(dimethylamino)propyl]carbodiimide hydrochloride (905 mg) were added. N-methylmorpholine (0.29 ml) was added to the mixture at 0°C, and the mixture was stirred for 7 hours at room temperature. The reaction mixture was poured into 1N hydrochloric acid, and the solution was extracted with ethyl acetate. The extract was washed with IN hydrochloric acid, a saturated aqueous solution of sodium bicarbonate, water and a solution aqueous solution of sodium chloride, successively, dried over anhydrous magnesium sulfate and concentrated. The residue was purified by column chromatography on silica gel (hexane : ethyl acetate = 5 : 1 → 3 : 1) to give the title compound (813 mg) having the following physical data.
TLC: Rf 0.32 (hexane : acetyl acetate = 3 : 1);
NMR ($CDCl_3$): δ 8.18 (d, J = 5.4 Hz, 1H), 6.78 (d, J = 8.1 Hz, 1H), 6.71-6.63 (m, 2H), 5.90 (d, J = 5.4 Hz, 1H), 4.38 (d, J = 3.0 Hz, 1H), 4.32-4.20 (m, 1H), 3.74 (s, 3H), 2.40-2.18 (br, 1H), 2.28 (s, 3H), 1.68 and 1.64 (each s, each 3H), 1.46 (s, 9H), 0.96 and 0.94 (each d, J = 6.9 Hz, each 3H).

Reference example 17

N-(t-butoxycarbonyl)-2,2-dimethyl-(4S)-(2-propyl)-(5R)-[3-(4-methoxy-2-methylphenyl)-1,3,4-oxadiazolin-2-one-5-yl]-1,3-oxazolidine

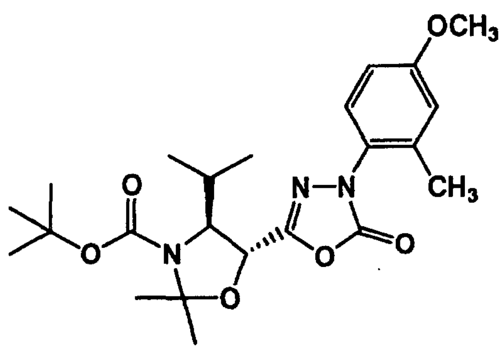

To a solution of the compound prepared in Reference example 16 (804 mg) in tetrahydrofuran (19 ml), under an argon atmosphere, triethylamine (0.80 ml) and 1,1'-carbodiimidazole (1.54 g) were added. The mixture was stirred for 15 hours at 80°C. The reaction mixture was poured into 1N hydrochloric acid, and the solution was extracted with ethyl acetate. The extract was washed with a saturated aqueous solution of sodium bicarbonate, water and a saturated aqueous solution of sodium chloride, dried over anhydrous magnesium sulfate and concentrated. The residue was purified by column chromatography on silica gel (hexane : ethyl acetate = 9 : 1) to give the title compound (501 mg) having the following physical data.

TLC: Rf 0.42 (hexane : acetyl acetate = 3 : 1);
NMR ($CDCl_3$): δ 7.25 (d, J = 9.0 Hz, 1H), 6.85-6.76 (m, 2H), 4.82 (d, J = 3.0 Hz, 1H), 4.50-4.27 (br, 1H), 3.82 (s, 3H), 2.50-2.14 (br, 1H), 2.25 (s, 3H), 1.62 and 1.60 (each s, each 3H), 1.48 (s, 9H), 0.98 and 0.95 (each d, J = 6.0 Hz, each 3H).

Reference example 18

(1S)-[(3-(4-methoxy-2-methylphenyl)-1,3,4-oxadiazolin-2-one-5-yl)-(1R)-hydroxymethyl]-2-methylpropylamine p-toluenesulfonate

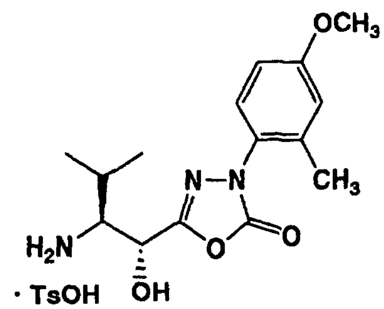

To a solution of the compound prepared in Reference example 17 (496 mg) in ethanol (23 ml), p-toluenesulfonic acid hydrate (294 mg) was added. The mixture was stirred overnight at 80°C. The reaction mixture was concentrated to give the title compound (662 mg) having the following physical data.
TLC: Rf 0.18 (chloroform : methanol = 9 : 1);
NMR (DMSO-$d_6$): δ 7.98 (br, 3H), 7.49 (d, J = 8.0 Hz, 2H), 7.46-7.02 (m, 4H), 7.00-6.84 (m, 2H), 4.89 (d, J = 4.0 Hz, 1H), 3.79 (s, 3H), 3.45-3.22 (m, 1H), 2.29 (s, 3H), 2.23 (s, 3H), 2.19-1.88 (m, 1H), 1.03 and 0.98 (each d, J = 7.0 Hz, each 3H).

Reference example 19

(methoxycarbonyl)-L-valyl-N-[(1S)-([3-(4-methoxy-2-methylphenyl)-1,3,4-oxadiazolin-2-one-5-yl]-(1R)-hydroxymethyl-2-methylpropyl]-L-prolinamide

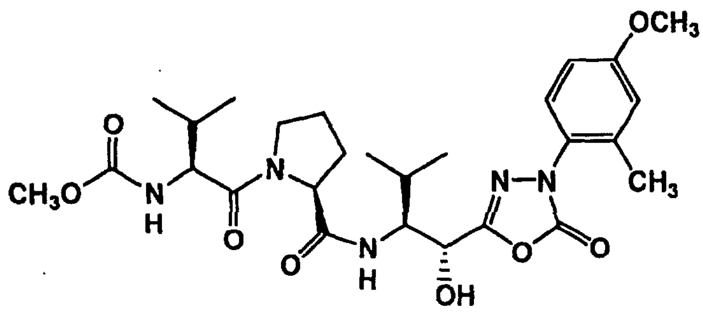

To a solution of the compound prepared in Reference example 18 (296 mg) and (methoxycarbonyl)-L-valyl-L-proline (136 mg) in demethylformamide (5 ml), under an argon atmosphere, 1-hydroxybenzotriazole hydrate (92 mg), 1-ethyl-3-[3-(dimethylamino)propyl]carbodiimide hydrochloride (115 mg) and 4-methylmorpholine (0.11 ml) were added. The mixture was stirred for 4 hours at room temperature. The reaction mixture was poured into 1N hydrochloric acid, and the solution was extracted with ethyl acetate. The extract was washed with a saturated aqueous solution of sodium bicarbonate, water and a saturated aqueous solution of sodium chloride, dried over anhydrous magnesium

sulfate and concentrated. The residue was purified by column chromatography on silica gel (chloroform : methanol = 1 : 0 → 99 : 1) to give the title compound (272 mg) having the following physical data.
TLC: Rf 0.40 (chloroform : methanol = 9 : 1);
NMR (DMSO-$d_6$): δ 7.81 (d, J = 9.3 Hz, 1H), 7.25 (d, J = 8.4 Hz, 1H), 7.23 (d, J = 8.6 Hz, 1H), 6.94 (d, J = 3.0 Hz, 1H), 6.88 (dd, J = 8.6 and 3.0 Hz, 1H). 6.08 (d, J = 5.4 Hz, 1H), 4.74 (dd, J = 5.4 and 3.0 Hz, 1H), 4.39 (dd, J =8.4 and 4.2 Hz, 1H), 4.07-3.90, 3.77-3.62 and 3.62-3.40 (each m, total 4H), 3.79 (s, 3H), 3.51 (s, 3H), 2.17 (s, 3H), 2.08-1.66 (m, 6H), 0.97, 0.91, 0.90 and 0.86 (each d, J = 6.9 Hz, each 3H).

Example 3

(methoxycarbonyl)-L-valyl-N-[(1S)-([3-(4-methoxy-2-methylphenyl)-1,3,4-oxadiazolin-2-one-5-yl]carbonyl)-2-methylpropyl]-L-prolinamide

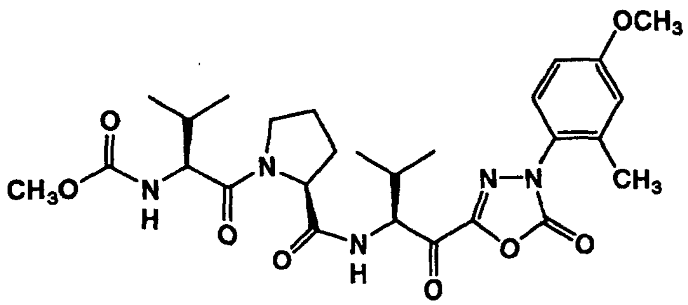

To a solution of oxalyl chloride (84 µl) in dichloromethane (3 ml), under an argon atmosphere, a solution of 1M dimethylsulfoxide in dichloromethane (1.92 ml) was dropped slowly at -78°C. The mixture was stirred for 30 minutes at -78°C. A solution of the compound prepared in Reference example 19 (268 mg) in dichloromethane (3 ml) was added to the mixture at -78°C. After the mixture was stirred for 2.5 hours at same temperature, 4-methylmorpholine (0.42 ml) was added to the mixture at -78°C. The mixture was stirred for 1 hour at -20°C. To a reaction mixture, IN hydrochloric acid was added, and the solution was extracted with ethyl acetate. The extract was washed with a saturated aqueous solution of sodium chloride, dried over magnesium chloride and concentrated. The residue was purified by column chromatography on silica gel (chloroform : methanol = 1 : 0 → 99 : 1) to give the title compound (241 mg) having the following physical data.
TLC: Rf 0.25 (hexane : ethyl acetate = 1 : 3);
NMR ($CDCl_3$): δ 7.46 (d, J = 7.1 Hz, 1H), 7.32-7.25 (m, 1H), 6.88-6.79 (m, 2H), 5.36 (d, J = 9.5 Hz, 1H), 5.14 (dd, J = 7.1 and 5.7 Hz, 1H), 4.63 (dd, J = 8.4 and 3.0 Hz, 1H), 4.31 (dd, J = 9.5 and 6.9 Hz, 1H), 3.85-3.53 (m, 2H), 3.84 (s, 3H), 3.68 (s, 3H), 2.44-1.82 (m, 6H), 2.28 (s, 3H), 1.03, 0.99, 0.95 and 0.94 (each d, J = 6.9 Hz, each 3H).

Reference example 20

N-(t-butoxycarbonyl)-2, 2-dimethyl-(4S)-(2-propyl)-(5R)-[3-(4-nitrophenyl)-1,3, 4-oxadiazolin-2-one-5-yl]-1,3-oxazolidine

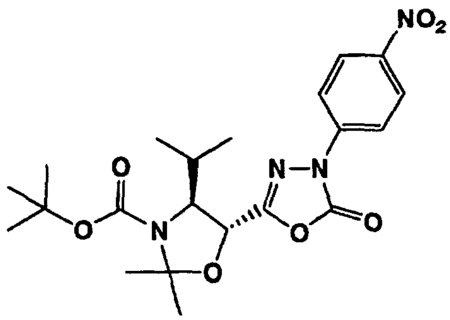

The title compound having the following physical data was obtained by the same procedure as a series of reaction of Reference example 16 → Reference example 17, using 4-nitrophenylhydrazine in stead of 4-methoxy-2-methylphenylhydrazine.
TLC: Rf 0.68 (hexane : ethyl acetate = 2 : 1);
NMR ($CDCl_3$): δ 8.34 and 8.09 (each d, J = 9.6 Hz, each 2H), 4.87 (d, J = 3.0 Hz, 1H), 4.57-4.19 (br, 1H), 2.54-2.14 (br, 1H), 1.63 and 1.61 (each s, each 3H), 1.50 (s, 9H), 1.00 and 0.98 (each d, J = 6.8 Hz, each 3H).

Reference example 21

N-(t-butoxycarbonyl)-2, 2-dimethyl-(4S)-(2-propyl)-(5R)-[3-(4-aminophenyl)-1, 3,4-oxadiazolin-2-one-5-yl]-1,3-oxazolidine

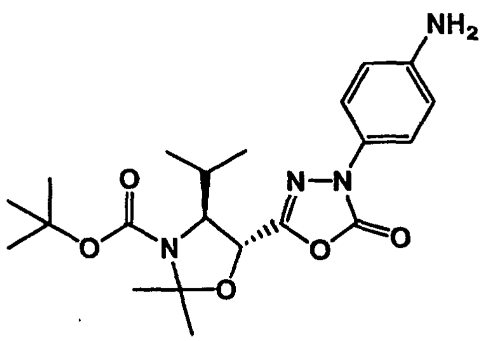

To a solution of the compound prepared in Reference example 20 (350 mg) in methanol (2.3 ml) and dioxane (4.6 ml), 20% palladium hydroxide (70 mg) was added. The mixture was stirred for 2.5 hours at room temperature under hydrogen gas atmosphere. The reaction mixture was filtered and the filtrate was concentrated to give the title compound (353 mg) having the following physical data.
TLC: Rf 0.13 (hexane : ethyl acetate = 3 : 1);
NMR ($CDCl_3$): δ 7.56 and 6.73 (each d, J = 8.7 Hz, each 2H), 4.82 (d, J = 3.0 Hz, 1H), 4.60-4.15 (br, 1H), 2.52-2.16 (br, 1H), 1.61 and 1.59 (each s, each 3H), 1.49 (s, 9H), 0.98 and 0.96 (each d, J = 6.6 Hz, each 3H).

Reference example 22

N-(t-butoxycarbonyl)-2,2-dimethyl-(4S)-(2-propyl)-(5R)-[3-(4-dimethylaminophenyl)-1,3,4-oxadiazolin-2-one-5-yl]-1,3-oxazolidine

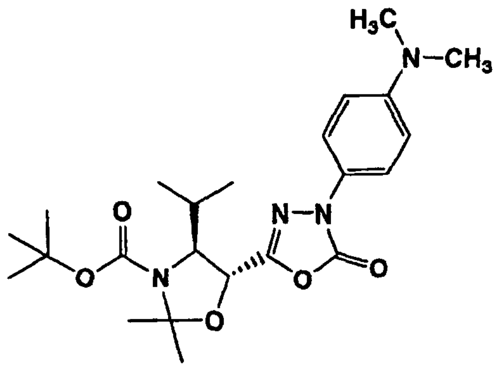

To a solution of the compound prepared in Reference example 21 (340 mg) in acetonitrile (15 ml), 35% aqueous solution of formaldehyde (0.4 ml) and sodium cyanoborohydride (78 mg) were added. The mixture was stirred for 30 minutes at room temperature. A reaction mixture was acidified by adding acetic acid to pH 4 -pH 5, and the mixture was stirred for 1 hour at room temperature. A saturated aqueous solution of sodium bicarbonate was added to the reaction mixture, and then a mixture was extracted with ethyl acetate. The extract was washed with water and a saturated aqueous solution of sodium chloride, dried over magnesium sulfate and concentrated. The residue was purified by column chromatography on silica gel (hexane : ethyl acetate = 9 : 1) to give the title compound (219 mg) having the following physical data.
TLC: Rf 0.47 (hexane : ethyl acetate = 3 : 1);
NMR ($CDCl_3$): δ 7.60 and 6.75 (each d, J = 9.0 Hz, each 2H), 4.83 (d, J = 2.7 Hz, 1H), 4.57-4.16 (br, 1H), 2.97 (s, 6H), 2.57-2.10 (br, 1H), 1.61 and 1.59 (each s, each 3H), 1.49 (s, 9H), 0.98 and 0.96 (each d, J = 6.6 Hz, each 3H).

Reference example 23

(methoxycarbonyl)-L-valyl-N-[(1S)-([3-(4-dimethylaminophenyl)-1,3,4-oxadiazolin-2-one-5-yl]-(1R)hydroxymethyl)-2-methylpropyl]-L-prolinamide

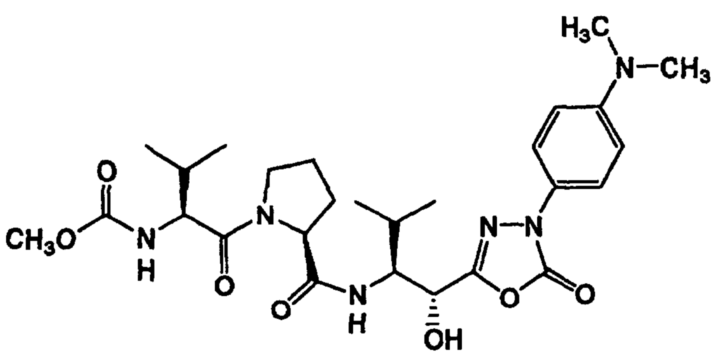

The title compound having the following physical data was obtained by the same procedure as a series of reaction of Reference example 18 → Reference example 19, using the compound prepared in Reference example 22 in stead of the compound prepared in Reference example 17.
TLC: Rf 0.34 (chloroform : methanol = 9 : 1);

NMR (DMSO-$d_6$): δ 7.83 (d, J = 9.6 Hz, 1H), 7.47 (d, J = 9.6 Hz, 2H), 7.21 (d, J = 8.4 Hz, 1H), 6.80 (d, J = 9.6 Hz, 2H), 6.10 (d, J = 5.4 Hz, 1H), 4.77 (dd, J = 5.4 and 2.4 Hz, 1H), 4.37 (dd, J =8.4 and 3.9 Hz, 1H), 4.05-3.88 and 3.82-3.36 (each m, total 4H), 3.50 (s, 3H), 2.92 (s, 6H), 2.02-1.62 (m, 6H), 0.98, 0.91, 0.87 and 0.84 (each d, J = 6.6 Hz, each 3H).

Example 4

(methoxycarbonyl)-L-valyl-N-[(1S)-([3-(4-dimethylaminophenyl)-1,3,4-oxadiazolin-2-one-5-yl]carbonyl)-2-methylpropyl]-L-prolinamide

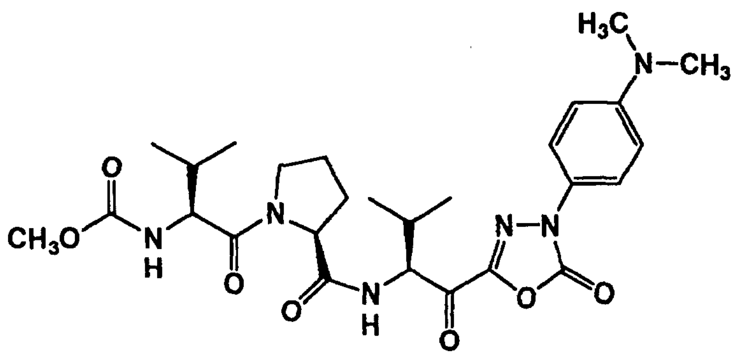

The title compound having the following physical data was obtained by the same procedure as Example 3, using the compound prepared in Reference example 23 in stead of the compound prepared in Reference example 19.
TLC: Rf 0.29 (hexane : ethyl acetate = 1 : 3);
NMR ($CDCl_3$): δ 7.64 (d, J = 9.2 Hz, 2H), 7.45 (d, J = 7.4 Hz, 1H), 6.75 (d, J = 9.2 Hz, 2H), 5.39 (d, J = 9.0 Hz, 1H), 5.23 (dd, J = 7.4 and 5.4 Hz, 1H), 4.63 (dd, J = 8.0 and 2.8 Hz, 1H), 4.32 (dd, J = 9.0 and 6.6 Hz, 1H), 3.84-3.52 (m, 2H), 3.68 (s, 3H), 3.00 (s, 6H), 2.45-1.79 (m, 6H), 1.04, 1.01, 0.97 and 0.95 (each d, J = 6.6 Hz, each 3H).

Example 5(1) - 5(18)

The following compounds of the present invention were obtained by the same procedure as a series of reaction of Reference Example 16 → Reference Example 17 → Reference Example 18 → Reference Example 19 → Example 3, using a corresponding hydrazine in stead of 4-methoxy-2-methylphenylhydrazine.

Example 5(1)

(methoxycarbonyl)-L-valyl-N-[(1S)-([3-(4-methoxyphenyl)-1,3,4-oxadiazolin-2-one-5-yl]carbonyl)-2-methylpropyl]-L-prolinamide

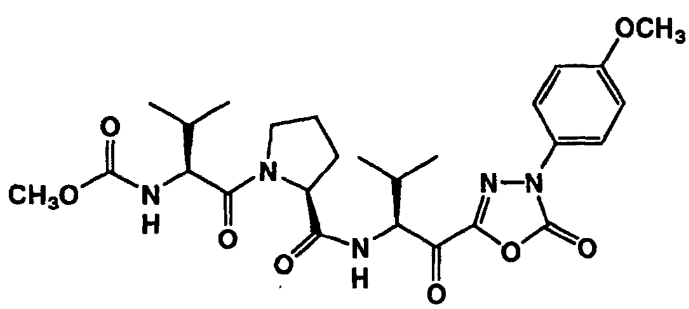

TLC: Rf 0.34 (hexane : ethyl acetate = 1 : 2);
NMR ($CDCl_3$): δ 7.76 (d, J = 9.3 Hz, 2H), 7.50 (d, J = 7.2 Hz, 1H), 6.99 (d, J = 9.3 Hz, 2H), 5.37 (d, J = 9.3 Hz, 1H), 5.21 (dd, J = 7.2 and 5.4 Hz, 1H), 4.63 (dd, J = 8.1 and 3.0 Hz, 1H), 4.31 (dd, J = 9.3 and 6.6 Hz, 1H), 3.85 (s, 3H), 3.83-3.57 (m, 2H), 3.68 (s, 3H), 2.40-1.83 (m, 6H), 1.05, 1.01, 0.97 and 0.96 (each d, J = 6.9 Hz, each 3H).

Example 5(2)

(methoxycarbonyl)-L-valyl-N-[(1S)-([3-(4-fluorophenyl)-1,3,4-oxadiazolin-2-one-5-yl]carbonyl)-2-methylpropyl]-L-prolinamide

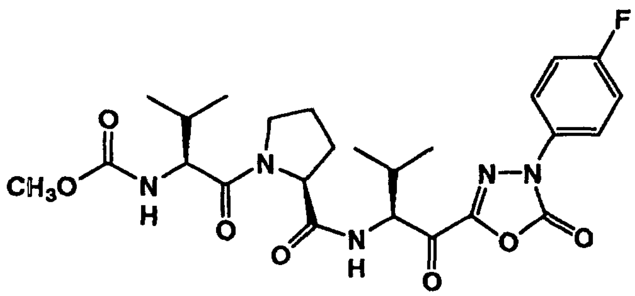

TLC: Rf 0.39 (hexane : ethyl acetate = 1 : 2);
NMR ($CDCl_3$): δ 7.87 (dd, J = 9.3, 4.5 Hz, 2H), 7.56 (d, J = 6.9 Hz, 1H), 7.18 (dd, J = 9.3, 8.4 Hz, 2H), 5.35 (d, J = 9.3 Hz, 1H), 5.18 (dd, J = 6.9, 5.7 Hz, 1H), 4.63 (dd, J = 7.8, 2.7 Hz, 1H), 4.32 (dd, J = 9.3, 6.9 Hz, 1H), 3.82-3.56 (m, 2H), 3.68 (s, 3H), 2.40-1.83 (m, 6H), 1.05, 1.01 and 0.97 (each d, J = 6.9 Hz, totally 12H).

Example 5(3)

(methoxycarbonyl)-L-valyl-N-[(1S)-([3-(4-methylphenyl)-1,3,4-oxadiazolin-2-one-5-yl]carbonyl)-2-methylpropyl]-L-prolinamide

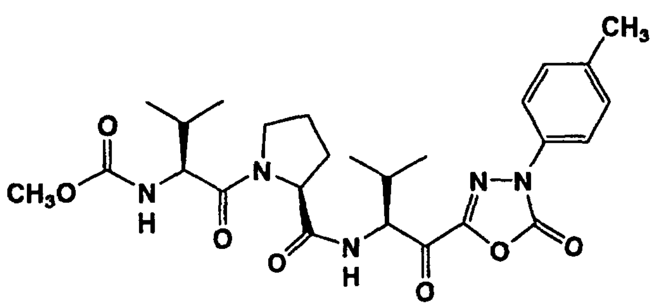

TLC: Rf 0.30 (hexane : ethyl acetate = 1: 2);
NMR ($CDCl_3$): δ 7.74 (d, J = 8.4 Hz, 2H), 7.51 (d, J = 7.2 Hz, 1H), 7.28 (d, J = 8.4 Hz, 2H), 5.37 (d, J = 9.3 Hz, 1H), 5.22 (dd, J = 7.2, 5.4 Hz, 1H), 4.63 (dd, J = 7.8, 2.7 Hz, 1H), 4.32 (dd, J = 9.3, 6.9 Hz, 1H), 3.82-3.55 (m, 2H), 3.68 (s, 3H), 2.39 (s, 3H), 2.38-1.83 (m, 6H), 1.05, 1.01, 0.97 and 0.96 (each d, J = 6.9 Hz, each 3H).

Example 5(4)

(methoxycarbonyl)-L-valyl-N-[(1S)-([3-(3-methylphenyl)-1,3,4-oxadiazolin-2-one-5-yl]carbonyl)-2-methylpropyl]-L-prolinamide

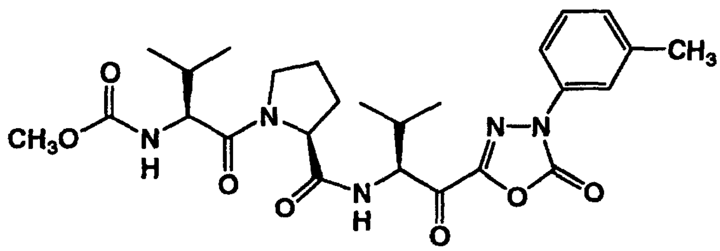

TLC: Rf 0.30 (hexane : ethyl acetate = 1 : 2);
NMR ($CDCl_3$): δ 7.69 (s, 1H), 7.68 (d, J = 7.5 Hz, 1H), 7.51 (d, J = 7.2 Hz, 1H), 7.36 (t, J = 7.5 Hz, 1H), 7.16 (d, J = 7.5 Hz, 1H), 5.37 (d, J = 9.0 Hz, 1H), 5.23 (dd, J = 7.2, 5.4 Hz, 1H), 4.63 (dd, J = 8.1, 3.0 Hz, 1H), 4.32 (dd, J = 9.0, 7.2 Hz, 1H), 3.85-3.55 (m, 2H), 3.68 (s, 3H), 2.43 (s, 3H), 2.40-1.83 (m, 6H), 1.06, 1.01, 0.97 and 0.96 (each d, J = 6.9 Hz, each 3H).

Example 5(5)

(methoxycarbonyl)-L-valyl-N-[(1S)-([3-(2-methylphenyl)-1,3,4-oxadiazolin-2-one-5-yl]carbonyl)-2-methylpropyl]-L-prolinamide

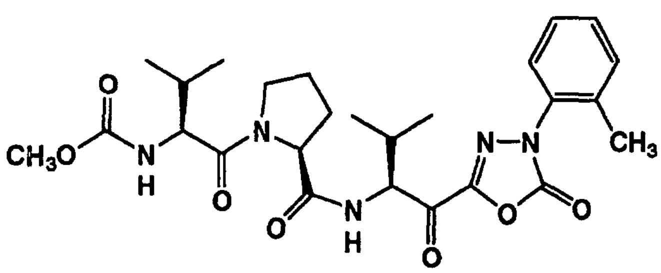

TLC: Rf 0.29 (hexane : ethyl acetate = 1 : 2);
NMR ($CDCl_3$): δ 7.48 (d, J = 7.2 Hz, 1H), 7.43-7.31 (m, 4H), 5.36 (d, J = 9.3 Hz, 1H), 5.15 (dd, J = 7.2, 5.4 Hz, 1H), 4.63 (dd, J = 8.4, 3.0 Hz, 1H), 4.31 (dd, J = 9.3, 6.9 Hz, 1H), 3.82-3.55 (m, 2H), 3.68 (s, 3H), 2.41-1.82 (m, 6H), 2.33 (s, 3H), 1.03, 0.99 and 0.95 (each d, J = 6.9 Hz, total 12H).

Example 5(6)

(methoxycarbonyl)-L-valyl-N-[(1S)-([3-(4-chlorophenyl)-1,3,4-oxadiazolin-2-one-5-yl]carbonyl)-2-methylpropyl]-L-prolinamide

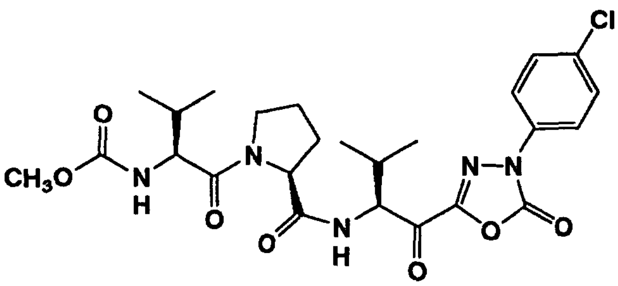

TLC: Rf 0.30 (hexane : ethyl acetate = 1 : 2);
NMR ($CDCl_3$): δ 7.86 (d, J = 9.3 Hz, 2H), 7.57 (d, J = 7.2 Hz, 1H), 7.46 (d, J = 9.3 Hz, 2H), 5.36 (d, J = 9.3 Hz, 1H), 5.17 (dd, J = 7.2, 6.0 Hz, 1H), 4.63 (dd, J = 8.1, 3.0 Hz, 1H), 4.32 (dd, J = 9.3, 6.6 Hz, 1H), 3.83-3.57 (m, 2H), 3.68 (s, 3H), 2.40-1.82 (m, 6H), 1.05, 1.01 and 0.97 (each d, J = 6.9 Hz, total 12H).

Example 5(7)

(methoxycarbonyl)-L-valyl-N-[(1S)-([3-(2, 4-difluorophenyl)-1,3,4-oxadiazolin-2-one-5-yl]carbonyl)-2-methylpropyl]-L-prolinamide

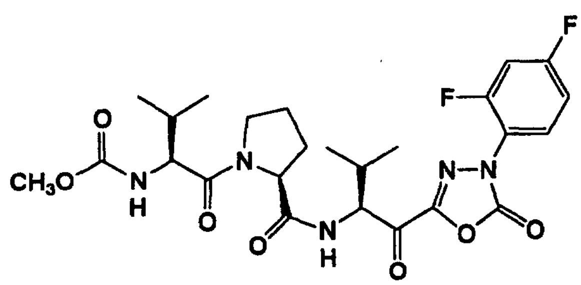

TLC: Rf 0.55 (chloroform : methanol = 10 : 1);
NMR (DMSO-$d_6$): δ 8.47 (d, J = 6.9 Hz, 1H), 7.84-7.77 (m, 1H), 7.65-7.58 (m, 1H). 7.37-7.29 (m, 1H), 7.25 (d, J = 8.4 Hz, 1H), 4.80 (t, J = 6.9 Hz, 1H), 4.43 (dd, J = 8.1, 4.5 Hz, 1H), 3.98 (t, J = 8.4 Hz, 1H), 3.77-3.68 and 3.58-3.50 (each m, totally 5H), 2.34-2.24 and 2.08-1.71 (each m, totally 6H), 0.96-0.83 (m, 12H).

Example 5(8)

(methoxycarbonyl)-L-valyl-N-[(1S)-([3-(4-cyanophenyl)-1,3,4-oxadiazolin-2-one-5-yl]carbonyl)-2-methylpropyl]-L-prolinamide

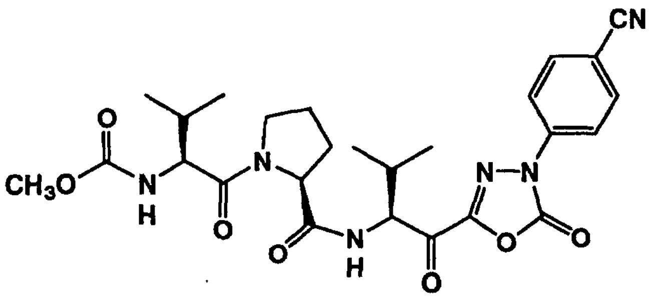

TLC: Rf 0.60 (chloroform : methanol = 10 : 1);
NMR (DMSO-$d_6$): δ 8.49 (d, J = 6.9 Hz, 1H), 8.10-7.98 (m, 4H), 7.21 (d, J = 8.4 Hz, 1H), 4.92 (t, J = 6.9 Hz, 1H), 4.47 (dd, J = 8.1, 4.8 Hz, 1H), 3.98 (t, J = 8.4 Hz, 1H), 3.78-3.68 and 3.58-3.50 (each m, totally 5H), 2.40-2.28 and 2.08-1.70 (each m, totally 6H), 1.00-0.83 (m, 12H).

Example 5(9)

(methoxycarbonyl)-L-valyl-N-[(1S)-([3-(4-trifluoromethylphenyl)-1,3,4-oxadiazolin-2-one-5-yl]carbonyl)-2-methylpropyl]-L-prolinamide

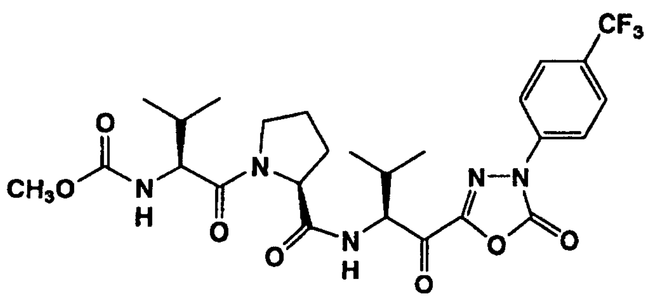

TLC: Rf 0.42 (hexane : ethyl acetate = 1 : 2);
NMR ($CDCl_3$): δ 8.08 (d, J = 8.7 Hz, 2H), 7.76 (d, J = 8.7 Hz, 2H), 7.63 (d, J = 6.6 Hz, 1H), 5.35 (d, J = 9.3 Hz, 1H), 5.16 (dd, J = 6.6, 5.7 Hz, 1H), 4.64 (dd, J = 8.4, 2.7 Hz, 1H), 4.32 (dd, J = 9.3, 6.9 Hz, 1H), 3.84-3.56 (m, 2H), 3.68 (s, 3H), 2.41-1.83 (m, 6H), 1.06, 1.01, 0.98 and 0.97 (each d, J = 6.9 Hz, each 3H).

Example 5(10)

(methoxycarbonyl)-L-valyl-N-[(1S)-([3-(4-trifluoromethoxyphenyl)-1,3,4-oxadiazolin-2-one-5-yl]carbonyl)-2-methylpropyl]-L-prolinamide

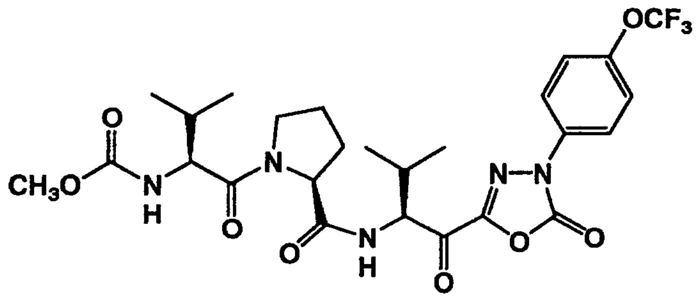

TLC: Rf 0.45 (hexane : ethyl acetate = 1 : 2);
NMR ($CDCl_3$): δ 7.96 (d, J = 9.0 Hz, 2H), 7.59 (d, J = 6.9 Hz, 1H), 7.34 (d, J = 9.0 Hz, 2H), 5.35 (d, J = 9.0 Hz, 1H), 5.16 (dd, J = 6.9, 5.7 Hz, 1H), 4.63 (dd, J = 8.1, 3.0 Hz, 1H), 4.32 (dd, J = 9.0, 6.6 Hz, 1H), 3.83-3.56 (m, 2H), 3.68 (s, 3H), 2.41-1.82 (m, 6H), 1.05, 1.01 and 0.97 (each d, J = 6.9 Hz, total 12H).

Example 5(11)

(methoxycarbonyl)-L-valyl-N-[(1S)-([3-(2-methoxyphenyl)-1,3,4-oxadiazolin-2-one-5-yl]carbonyl)-2-methylpropyl]-L-prolinamide

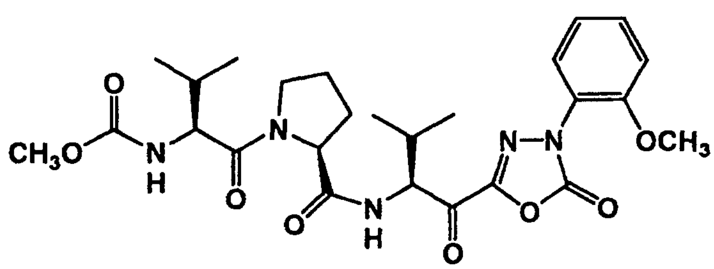

TLC: Rf 0.21 (hexane : ethyl acetate = 1 : 2);
NMR ($CDCl_3$): δ 7.52-7.35 (m, 3H), 7.11-7.01 (m, 2H), 5.37 (d, J = 9.2 Hz, 1H), 5.18 (dd, J = 7.2 and 5.4 Hz, 1H), 4.62 (dd, J = 8.4 and 3.0 Hz, 1H), 4.31 (dd, J = 9.2 and 7.2 Hz, 1H), 3.87 (s, 3H), 3.84-3.54 (m, 2H), 3.68 (s, 3H), 2.40-1.81 (m, 6H), 1.03, 0.99, 0.95 and 0.94 (each d, J = 6.9 Hz, each 3H).

Example 5(12)

(methoxycarbonyl)-L-valyl-N-[(1S)-([3-(2,4-dimethylphenyl)-1,3,4-oxadiazolin-2-one-5-yl]carbonyl)-2-methylpropyl] -L-prolinamide

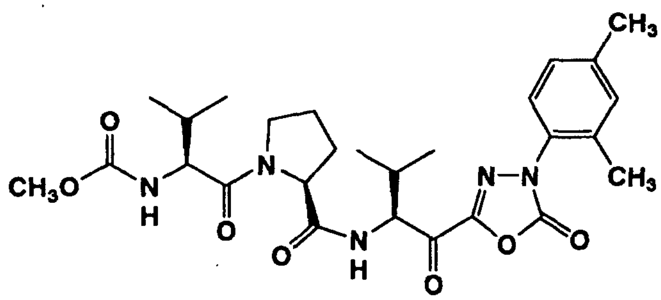

TLC: Rf 0.34 (hexane : ethyl acetate = 1 : 2);
NMR ($CDCl_3$): δ 7.47 (d, J = 7.2 Hz, 1H), 7.25 (d, J = 8.7 Hz, 1H), 7.15 (s, 1H), 7.12 (d, J = 8.7 Hz, 1H), 5.37 (d, J = 9.0 Hz, 1H), 5.15 (dd, J = 7.2, 5.4 Hz, 1H), 4.62 (dd, J = 8.4, 3.0 Hz, 1H), 4.31 (dd, J = 9.0, 6.6 Hz, 1H), 3.82-3.55 (m, 2H), 3.68 (s, 3H), 2.45-1.84 (m, 6H), 2.38 and 2.28 (each s, each 3H), 1.03, 0.99, 0.95 and 0.94 (each d, J = 6.9 Hz, each 3H).

Example 5(13)

(methoxycarbonyl)-L-valyl-N-[(1S)-([3-(2-trifluoromethylphenyl)-1,3,4-oxadiazolin-2-one-5-yl]carbonyl)-2-methylpropyl]-L-prolinamide

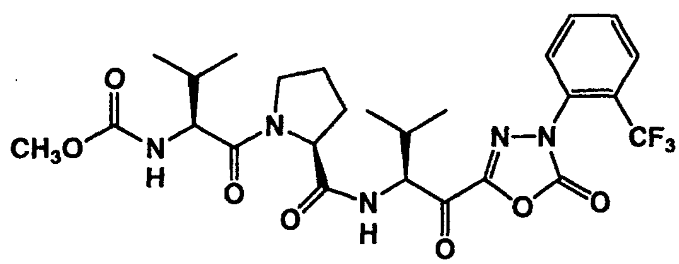

TLC: Rf 0.25 (hexane : ethyl acetate = 1 : 3);
NMR ($CDCl_3$): δ 7.86 (dd, J = 7.2 and 1.2 Hz, 1H), 7.80-7.58 (m, 3H), 7.51 (d, J = 6.8 Hz, 1H), 5.35 (d, J = 9.3 Hz, 1H), 5.06 (dd, J = 6.8 and 5.7 Hz, 1H), 4.63 (dd, J = 7.8 and 2.7 Hz, 1H), 4.30 (dd, J = 9.3 and 6.9 Hz, 1H), 3.83-3.51 (m, 2H), 3.67 (s, 3H), 2.42-1.80 (m, 6H), 1.09-0.87 (m, 12H).

Example 5(14)

(methoxycarbonyl)-L-valyl-N-[(1S)-([3-(4-ethoxyphenyl)-1,3,4-oxadiazolin-2-one-5-yl]carbonyl)-2-methylpropyl]-L-prolinamide

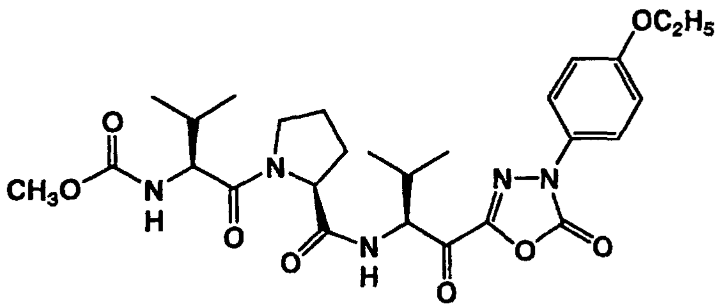

TLC: Rf 0.32 (hexane : ethyl acetate = 1: 2);
NMR ($CDCl_3$): δ 7.74 (d, J = 9.3 Hz, 2H), 7.50 (d, J = 6.9 Hz, 1H), 6.97 (d, J = 9.3 Hz, 2H), 5.37 (d, J = 9.3 Hz, 1H), 5.21 (dd, J = 6.9, 5.4 Hz, 1H), 4.63 (dd, J = 8.1, 3.0 Hz, 1H), 4.32 (dd, J = 9.3, 6.9 Hz, 1H), 4.07 (q, J = 6.9 Hz, 2H), 3.82-3.57 (m, 2H), 3.68 (s, 3H), 2.40-1.82 (m, 6H), 1.44 (t, J = 6.9 Hz, 3H), 1.05, 1.01, 0.97 and 0.96 (each d, J = 6.9 Hz, each 3H).

Example 5(15)

(methoxycarbonyl)-L-valyl-N-[(IS)-([3-(2,4-dimethoxyphenyl)-1,3,4-oxadiazolin-2-one-5-yl]carbonyl)-2-methylpropyl] -L-prolinamide

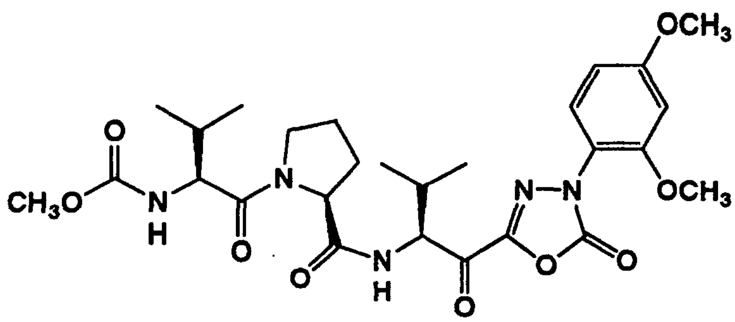

TLC: Rf 0.22 (hexane : ethyl acetate = 1 : 3);
NMR ($CDCl_3$): δ 7.40 (d, J = 7.3 Hz, 1H), 7.31 (d, J = 9.2 Hz, 1H), 6.61-6.51 (m, 2H), 5.37 (d, J = 9.0 Hz, 1H), 5.18 (dd, J = 7.3 and 5.4 Hz, 1H), 4.62 (dd, J = 8.2 and 3.4 Hz, 1H), 4.31 (dd, J = 9.0 and 7.0 Hz, 1H), 3.91-3.48 (m, 2H), 3.86 and 3.83 (each s, each 3H), 3.68 (s, 3H), 2.46-1.77 (m, 6H), 1.02, 0.99, 0.95 and 0.93 (each d, J = 6.6 Hz, each 3H).

Example 5(16)

(methoxycarbonyl)-L-valyl-N-[(1S)-([3-(4-fluoro-2-methylphenyl)-1,3,4-oxadiazolin-2-one-5-yl]carbonyl)-2-methylpropyl]-L-prolinamide

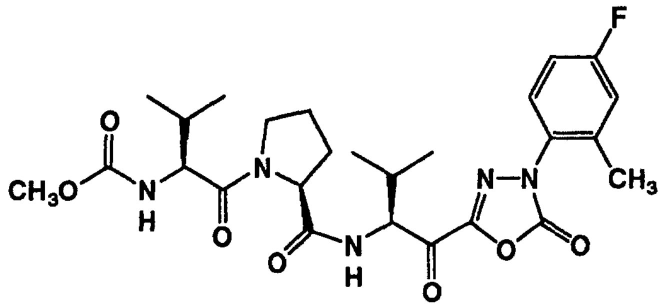

TLC: Rf 0.37 (hexane : ethyl acetate = 1 : 2);
NMR ($CDCl_3$): δ 7.51 (d, J = 6.9 Hz, 1H), 7.37 (dd, J = 8.7, 5.4 Hz, 1H), 7.09-6.98 (m, 2H), 5.35 (d, J = 9.3 Hz, 1H), 5.12 (dd, J = 6.9, 5.4 Hz, 1H), 4.63 (dd, J = 8.4, 3.0 Hz, 1H), 4.31 (dd, J = 9.3, 6.9 Hz, 1H), 3.82-3.55 (m, 2H), 3.68 (s, 3H), 2.41-1.83 (m, 6H), 2.32 (s, 3H), 1.03, 0.99 and 0.95 (each d, J = 6.9 Hz, total 12H).

Example 5(17)

(methoxycarbonyl)-L-valyl-N-[(1S)-([3-(4-fluoro-2-methoxyphenyl)-1,3,4-oxadiazolin-2-one-5-yl]carbonyl)-2-methylpropyl]-L-prolinamide

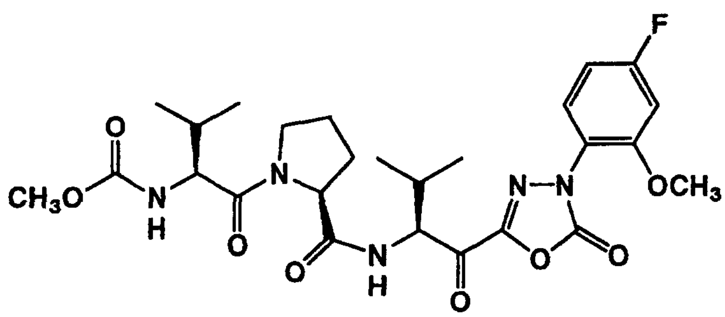

TLC: Rf 0.37 (hexane : ethyl acetate = 1 : 3);
NMR ($CDCl_3$): δ 7.44 (d, J = 7.5 Hz, 1H), 7.38 (dd, J = 9.2 and 5.9 Hz, 1H), 6.82-6.71 (m, 2H), 5.36 (d, J = 9.0 Hz, 1H), 5.15 (dd, J = 7.5 and 5.4 Hz, 1H), 4.62 (dd, J = 8.1 and 2.7 Hz, 1H), 4.31 (dd, J = 9.0 and 6.6 Hz, 1H), 3.86 (s, 3H), 3.85-3.54 (m, 2H), 3.68 (s, 3H), 2.40-1.83 (m, 6H), 1.03, 0.99, 0.95 and 0.94 (each d, J = 6.6 Hz, each 3H).

Example 5(18)

(methoxycarbonyl)-L-valyl-N-[(IS)-([3-(4-benzyloxyphenyl)-1,3,4-oxadiazolin-2-one-5-yl]carbonyl)-2-methylpropyl]-L-prolinamide

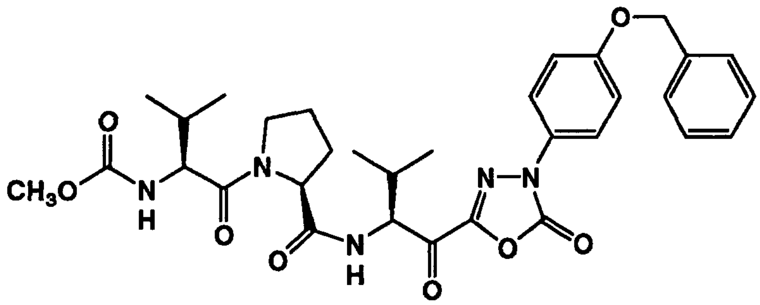

TLC: Rf 0.38 (hexane : ethyl acetate = 1 : 2);
NMR ($CDCl_3$): δ 7.76 (d, J = 9.3 Hz, 2H), 7.50 (d, J = 6.9 Hz, 1H), 7.47-7.30 (m, 5H), 7.05 (d, J = 9.3 Hz, 2H), 5.36 (d, J = 9.0 Hz, 1H), 5.19 (dd, J = 6.9, 5.7 Hz, 1H), 5.10 (s, 2H), 4.63 (dd, J = 8.1, 2.7 Hz, 1H), 4.32 (dd, J = 9.0, 6.6 Hz, 1H), 3.82-3.56 (m, 2H), 3.68 (s, 3H), 2.40-1.83 (m, 6H), 1.05, 1.01, 0.97 and 0.96 (each d, J = 6.9 Hz, each 3H).

Example 6

(methoxycarbonyl)-L-valyl-N-[(1S)-([3-(4-hydroxyphenyl)-1, 3, 4-oxadiazolin-2-one-5-yl]carbonyl)-2-methylpropyl]-L-prolinamide

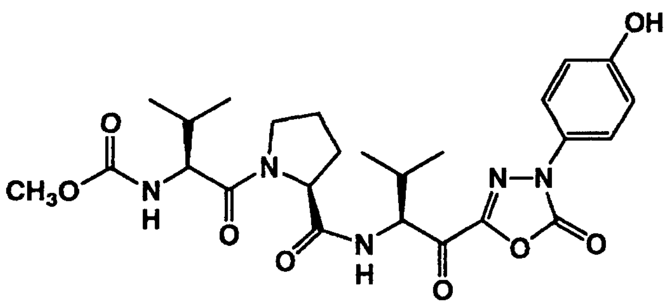

The compound prepared in Example 5(18) (338 mg) was dissolved into methanol (5.5 ml), and then under an argon atmosphere, the solution was deaerated. 10% Palladium-carbon (34 mg) was added to the solution, and then the mixture was stirred for 1 hour at room temperature, under hydrogen gas atmosphere. Under an argon atmosphere, 10% palladium-carbon (37 mg) was added, under hydrogen gas atmosphere, the mixture was stirred for 6 hours at room temperature. After the gaseous phase was replaced with argon, the reaction mixture was filtered through Celite (registered trademark). The filtrate was concentrated. The residue was purified by column chromatography on silica gel (chloroform → chloroform : methanol = 100 : 1) to give the title compound (283 mg) having the following physical data.
TLC: Rf 0.24 (hexane : acetyl acetate = 1 : 2);
NMR ($CDCl_3$): δ 7.61 (d, J = 9.3 Hz, 2H), 7.50 (d, J = 7.2 Hz, 1H), 6.89 (d, J = 9.3 Hz, 2H), 6.41 (s, 1H), 5.36 (d, J = 9.3 Hz, 1H), 5.20 (dd, J = 7.2, 5.4 Hz, 1H), 4.67-4.60 (m, 1H), 4.32 (dd, J = 9.3, 7.2 Hz, 1H), 3.85-3.57 (m, 2H), 3.68 (s, 3H), 2.39-1.86 (m, 6H), 1.05, 1.02 and 0.97 (each d, J = 6.9 Hz, total 12H).

Formulation example

Formulation example 1

The following components were admixed in conventional method and punched out to obtain 100 tablets each containing 50 mg of active ingredient.

- (methoxycarbonyl)-L-valyl-N-[(1S)-([3-phenyl-1,3,4-oxadiazolin-2-one-5-yl]carbonyl)-2-methylpropyl]-L-prolinamide 5.0 g
- Carboxymethylcellulose calcium (disintegrating agent) 0.2 g
- Magnesium stearate (lubricating agent) 0.1 g
- Microcrystaline cellulose 4.7 g

Formulation example 2

The following components were admixed in conventional method. The solution was sterilized in conventional manner, placed 5 ml portions into ampoules and freeze-dried to obtain 100 ampoules each containing 20 mg of the active ingredient.

- (methoxycarbonyl)-L-valyl-N-[(1S)-([3-phenyl-1,3,4-oxadiazolin-2-one-5-yl]carbonyl)-2-methylpropyl]-L-prolinamide 2.0 g
- Mannitol 20 g
- Distilled water 1000 ml

## Claims

**1.** 1, 3, 4-oxadiazolin-2-one derivatives of formula (I)

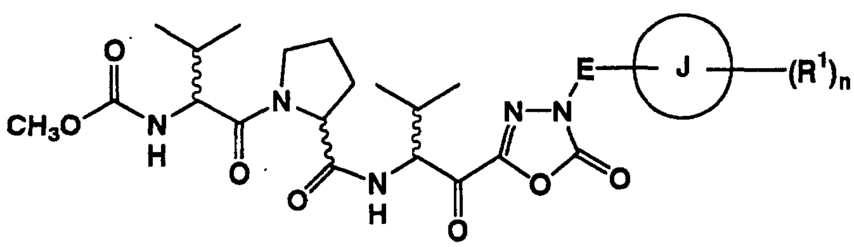

(I)

wherein E is a single bond or C1-8 alkylene,
J is C5-15 mono-, bi- or tri-carbacyclic ring or 5-18 membered mono-, bi- or tri-heterocyclic ring containing 1-4 of nitrogen, 1-2 of oxygen and/or 1-2 sulfur,

$R^1$ is

(1) C1-8 alkyl,
(2) halogen atom,
(3) nitro,
(4) trifluoromethyl,
(5) trifluoromethoxy,
(6) cyano,
(7) phenyl,
(8) tetrazolyl,
(9) $-NR^2R^3$,
(10) $-OR^4$,
(11) $-SR^5$,
(12) $-COR^6$ or
(13) C1-8 alkyl substituted by halogen atom, nitro, trifluoromethyl, trifluoromethoxy, cyano, phenyl, tetra-

zolyl, $-NR^2R^3$, $-OR^4$, $-SR^5$ or $-COR^6$, in which $R^2$, $R^3$, $R^4$ and $R^5$ each, independently, is hydrogen, C1-4 alkyl, phenyl or C1-4 alkyl substituted by phenyl, $R^6$ is C1-4 alkyl, phenyl, C1-4 alkyl substituted by phenyl, $-NR^2R^3$ or $-OR^4$,

n is 0 or 1-5;
or a non-toxic salt thereof.

**2.** The compound according to claim 1, which is selected from

(1) (methoxycarbonyl)-L-valyl-N-[(1S)-([3-(thiophen-3-ylmethyl)-1,3,4-oxadiazolin-2-one-5-yl]carbonyl)-2-methylpropyl]-L-prolinamide,
(2) (methoxycarbonyl)-L-valyl-N-[(1S)-([3-benzyl-1,3,4-oxadiazolin-2-one-5-yl]carbonyl)-2-methylpropyl]-L-prolinamide,
(3) (methoxycarbonyl)-L-valyl-N-[(1S)-([3-(2-phenylethyl)-1,3,4-oxadiazolin-2-one-5-yl]carbonyl)-2-methylpropyl]-L-prolinamide,
(4) (methoxycarbonyl)-L-valyl-N-[(1S)-([3-(3,4-methylenedioxyphenylmethyl)-1,3,4-oxadiazolin-2-one-5-yl]carbonyl)-2-methylpropyl]-L-prolinamide,
(5) (methoxycarbonyl)-L-valyl-N-[(1S)-([3-(thiophen-2-ylmethyl)-1,3,4-oxadiazolin-2-one-5-yl]carbonyl)-2-methylpropyl]-L-prolinamide,
(6) (methoxycarbonyl)-L-valyl-N-[(1S)-([3-(4-fluorophenylmethyl)-1,3,4-oxadiazolin-2-one-5-yl]carbonyl)-2-methylpropyl]-L-prolinamide,
(7) (methoxycarbonyl)-L-valyl-N-[(1S)-([3-phenyl-1,3,4-oxadiazolin-2-one-5-yl]carbonyl)-2-methylpropyl]-L-prolinamide,
(8) (methoxycarbonyl)-L-valyl-N-[(1S)-([3-(4-methoxy-2-methylphenyl)-1,3,4-oxadiazolin-2-one-5-yl]carbonyl)-2-methylpropyl]-L-prolinamide,
(9) (methoxycarbonyl)-L-valyl-N-[(1S)-([3-(4-dimethylaminophenyl)-1,3,4-oxadiazolin-2-one-5-yl]carbonyl)-2-methylpropyl]-L-prolinamide,
(10) (methoxycarbonyl)-L-valyl-N-[(1S)-([3-(4-methoxyphenyl)-1,3,4-oxadiazolin-2-one-5-yl]carbonyl)-2-methylpropyl]-L-prolinamide,
(11) (methoxycarbonyl)-L-valyl-N-[(1S)-([3-(4-fluorophenyl)-1,3,4-oxadiazolin-2-one-5-yl]carbonyl)-2-methylpropyl]-L-prolinamide,
(12) (methoxycarbonyl)-L-valyl-N-[(1S)-([3-(4-methylphenyl)-1,3,4-oxadiazolin-2-one-5-yl]carbonyl)-2-methylpropyl]-L-prolinamide,
(13) (methoxycarbonyl)-L-valyl-N-[(1S)-([3-(3-methylphenyl)-1,3,4-oxadiazolin-2-one-5-yl]carbonyl)-2-methylpropyl]-L-prolinamide,
(14) (methoxycarbonyl)-L-valyl-N-[(1S)-([3-(2-methylphenyl)-1,3,4-oxadiazolin-2-one-5-yl]carbonyl)-2-methylpropyl]-L-prolinamide,
(15) (methoxycarbonyl)-L-valyl-N-[(1S)-([3-(4-chlorophenyl)-1,3,4-oxadiazolin-2-one-5-yl]carbonyl)-2-methylpropyl]-L-prolinamide,
(16) (methoxycarbonyl)-L-valyl-N-[(1S)-([3-(2, 4-difluorophenyl)-1,3,4-oxadiazolin-2-one-5-yl]carbonyl)-2-methylpropyl]-L-prolinamide,
(17) (methoxycarbonyl)-L-valyl-N-[(1S)-([3-(4-cyanophenyl)-1,3,4-oxadiazolin-2-one-5-yl]carbonyl)-2-methylpropyl]-L-prolinamide,
(18) (methoxycarbonyl)-L-valyl-N-[(1S)-([3-(4-trifluoromethylphenyl)-1,3,4-oxadiazolin-2-one-5-yl]carbonyl)-2-methylpropyl]-L-prolinamide,
(19) (methoxycarbonyl)-L-valyl-N-[(1S)-([3-(4-trifluoromethoxyphenyl)-1,3,4-oxadiazolin-2-one-5-yl]carbonyl)-2-methylpropyl]-L-prolinamide,
(20) (methoxycarbonyl)-L-valyl-N-[(1S)-([3-(2-methoxyphenyl)-1,3,4-oxadiazolin-2-one-5-yl]carbonyl)-2-methylpropyl]-L-prolinamide,
(21) (methoxycarbonyl)-L-valyl-N-[(1S)-([3-(2, 4-dimethylphenyl)-1,3,4-oxadiazolin-2-one-5-yl]carbonyl)-2-methylpropyl]-L-prolinamide,
(22) (methoxycarbonyl)-L-valyl-N-[(1S)-([3-(2-trifluoromethylphenyl)-1,3,4-oxadiazolin-2-one-5-yl]carbonyl)-2-methylpropyl]-L-prolinamide,
(23) (methoxycarbonyl)-L-valyl-N-[(1S)-([3-(4-ethoxyphenyl)-1,3,4-oxadiazolin-2-one-5-yl]carbonyl)-2-methylpropyl]-L-prolinamide,
(24) (methoxycarbonyl)-L-valyl-N-[(1S)-([3-(2,4-dimethoxyphenyl)-1,3,4-oxadiazolin-2-one-5-yl]carbonyl)-2-methylpropyl]-L-prolinamide,
(25) (methoxycarbonyl)-L-valyl-N-[(1S)-([3-(4-fluoro-2-methylphenyl)-1,3,4-oxadiazolin-2-one-5-yl]carbonyl)-2-methylpropyl]-L-prolinamide,
(26) (methoxycarbonyl)-L-valyl-N-[(1S)-([3-(4-fluoro-2-methoxyphenyl)-1,3,4-oxadiazolin-2-one-5-yl]carbonyl)-2-methylpropyl]-L-prolinamide,
(27) (methoxycarbonyl)-L-valyl-N-[(1S)-([3-(4-benzyloxyphenyl)-1,3,4-oxadiazolin-2-one-5-yl]carbonyl)-2-methylpropyl]-L-prolinamide or
(28) (methoxycarbonyl)-L-valyl-N-[(1S)-([3-(4-hydroxyphenyl)-1,3,4-oxadiazolin-2-one-5-yl]carbonyl)-2-methylpropyl]-L-prolinamide.

**3.** Elastase inhibitors comprising 1, 3, 4-oxadiazolin-2-one derivatives of formula (I) or a non-toxic salt thereof described in claim 1, as active ingredient, and a pharmaceutically acceptable carrier.

**4.** 1, 3, 4-oxadiazolin-2-one derivatives of formula (I) or a non-toxic salt thereof described in claim 1 for use in the treatment and/or prevention of a disease induced by an abnormal enhancement of degradation of elastin, collagen fiber and / or proteoglycans by elastase.

**5.** 1, 3, 4-oxadiazolin-2-one derivatives of formula (I) or a non-toxic salt thereof for use in the treatment and/or prevention a disease according to claim 4, wherein a disease is selected from pulmonary emphysema, chronic obstructive pulmonary disease, rheumatoid arthritis, arteriosclerosis, adult respiratory distress syndrome, glomerular nephritis, myocardial infarction, ulcerative colitis and gingivitis.

# INTERNATIONAL SEARCH REPORT

International application No.
PCT/JP00/08516

A. CLASSIFICATION OF SUBJECT MATTER
Int.Cl7 C07K5/083, A61K38/06, A61P1/02, A61P1/04, A61P9/10, A61P11/00, A61P29/00, A61P43/00

According to International Patent Classification (IPC) or to both national classification and IPC

B. FIELDS SEARCHED

Minimum documentation searched (classification system followed by classification symbols)
Int.Cl7 C07K5/083, A61K38/06, A61P1/02, A61P1/04, A61P9/10, A61P11/00, A61P29/00, A61P43/00

Documentation searched other than minimum documentation to the extent that such documents are included in the fields searched

Electronic data base consulted during the international search (name of data base and, where practicable, search terms used)
CA(STN),REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | WO, 98/24806, A2 (CORTECH,INC.), 11 June, 1998 (11.06.98) & JP, 10-511933, A & US, 5618792, A | 1-5 |
| A | Maciej Wieczorek et al. "Biochemical Characterization of α-Ketooxadiazole Inhibitors of Elastases" Archives of Biochemistry and Biophysics (1999) Vol.367 No.2 P.193-201 | 1-5 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 22 February, 2001 (22.02.01) | 06 March, 2001 (06.03.01) |
| Name and mailing address of the ISA/ Japanese Patent Office | Authorized officer |
| Facsimile No. | Telephone No. |

Form PCT/ISA/210 (second sheet) (July 1992)